**Europäisches Patentamt**

**European Patent Office**

**Office européen des brevets**

(11) Veröffentlichungsnummer: **0 136 256**
A2

## (12) EUROPÄISCHE PATENTANMELDUNG

(21) Anmeldenummer: 84810137.4

(22) Anmeldetag: 23.03.84

(51) Int. Cl.⁴: **C 12 P 1/06**, C 12 P 19/28,
C 07 H 17/02, A 23 K 1/17
// (C12P1/06, C12R1:57),
(C12P19/28,C12R1:57)

(30) Priorität: 28.09.83 DD 255165
25.10.83 DD 255943
25.10.83 DD 255945
25.10.83 DD 255946
25.10.83 DD 255944

(43) Veröffentlichungstag der Anmeldung: 03.04.85
Patentblatt 85/14

(84) Benannte Vertragsstaaten: **AT BE DE FR GB NL**

(71) Anmelder: **VEB Jenapharm, Otto-Schott-Strasse 13, DDR-6900 Jena (DD)**

(72) Erfinder: **Bergter, Friedrich, Prof. Dr. Dipl. Biol., Rheinlandstrasse 36, DDR-6900 Jena (DD)**
Erfinder: **Bocker, Harald, Dr. Dipl. Biol., Steingraben, DDR-6900 Jena (DD)**
Erfinder: **Bormann, Ernst-Joachim, Dr. Dipl. Biol., Edwin-Morgner-Strasse 44, DDR-6902 Jena-Lobeda (DD)**
Erfinder: **Forberg, Wolfgang, Dr. Dipl. Chem., Hermann-Löns-Strasse 52, DDR-6900 Jena (DD)**
Erfinder: **Fricke, Heinz, Dr. Dipl. Chem., Emil-Höllein-Strasse 88, DDR-6900 Jena (DD)**
Erfinder: **Gräfe, Udo, Dr. Dipl. Chem., Hermann-Löns-Strasse 43b, DDR-6900 Jena (DD)**

(72) Erfinder: **Grosse, Hans-Helmut, Dr. Dipl. Phys., Felsenkellerstrasse 5, DDR-6900 Jena (DD)**
Erfinder: **Heller, Ingeborg, Dr. Dipl. Biol., Naumburger Strasse 25, DDR-6900 Jena (DD)**
Erfinder: **Hilliger, Matthias, Dr. Dipl. Biol., Adolf-Scheile-Strasse 14, DDR-6900 Jena (DD)**
Erfinder: **Junne, Wolf Dipl. Ing., Spitzweidenweg 20/204, DDR-6900 Jena (DD)**
Erfinder: **Linde, Hellmut, Dr. Dipl. Chem., Am Gänseberg 2, DDR-6900 Jena (DD)**
Erfinder: **Menner, Michael, Dr. Dipl. Biol., Ammerbacher Strasse 106, DDR-6901 Ammerbach (DD)**
Erfinder: **Menzel, Klaus-Dieter, Hermann-Matern-Strasse 13, DDR-5320 Apolda (DD)**
Erfinder: **Müller, Peter-Jürgen, Dr. Dipl. Chem., Merzenbergweg 1, DDR-6901 Ammerbach (DD)**
Erfinder: **Pionka, Gunter, Dr. Dipl. Biol., Strasse des 8. Mai 2, DDR-6900 Jena (DD)**
Erfinder: **Pohl, Hans Dieter, Dr. Dipl. Ing., von-Hase-Weg 14, DDR-6900 Jena (DD)**
Erfinder: **Schneider, Jörg, Prof. Dr. Dipl. Chem., Otto-Millitzer-Strasse 33, DDR-6902 Jena-Lobeda (DD)**
Erfinder: **Thrum, Heinz, Dr. Dipl. Chem., Hermann-Löns-Strasse 62, DDR-6900 Jena (DD)**

(74) Vertreter: **Becher, Pauline, Dr. et ai, A. Braun, Braun, Héritier, Eschmann AG Hoibeinstrasse 36-38, CH-4051 Basel (CH)**

(54) **Verfahren zur Herstellung von Nourseothricin und seiner Adsorbate.**

(57) Es wird ein Verfahren zur fermentativen Herstellung des ergotrop wirkenden Antibiotikums Nourseothricin beschrieben, mit dem unter Verwendung verschiedener Kohlenhydratquellen und geeigneten anorganischen und organischen Stickstoffquellen sowie verschiedener Mineralsalze mit oder ohne Zusatz von Hemmstoffen der Atmungskette oder des intrazellulären Aminosäuretransports über eine Beeinflussung des Phosphatstoffwechsels und Vermeidung weiterer Limitationen bei geregelten Azididätsbedingungen hohe Konzentrationen an diesem Antibiotikum in der Kulturlösung angereichert werden. Die Gewinnung des Wirkstoffs aus der Kulturlösung erfolgt entweder mittels chromatografischer Verfahren in Form von Salzen oder durch Zusatz eines physiologisch verträglichen Adsorbens als myzelhaltiges Nourseothricin-Adsorbat, welches bevorzugt zur Dotierung von Mischfuttermitteln eingesetzt werden kann.

Verfahren zur Herstellung von Nourseothricin und
seiner Adsorbate

Die Erfindung betrifft ein neues Verfahren zur fermentativen Herstellung von Nourseothricin und dessen Gewinnung in Form seiner Salze und Adsorbate.

Nourseothricin ist ein zur Gruppe der Streptothricine
gehörenden Antibiotikum, welches nach Verabreichung in
ergotropen Dosen mit dem Tierfutter bei landwirtschaftlichen Nutztieren eine beschleunigte Lebendmasse-Zunahme
sowie gleichzeitig eine Verminderung des Futteraufwandes
bewirkt. Es ist somit für die Tierproduktion sowie die
pharmazeutische und die Mischfuttermittelindustrie von
Nutzen.

Das aus der Kultur einer Variante von Streptomyces
noursei ATCC 11455 isolierte Antibiotikum Nourseothricin ist in vitro wirksam gegen Gram-positive und -negative Bakterien sowie gegen Mykobakterien (Bradler, G.
und Thrum, H.: Nourseothricin A und B, zwei neue antibakterielle Antibiotika einer Streptomyces-Noursei-Variante. Zschr. Allgem. Mikrobiol. $\underline{3}$, 105 bis 112 (1963)).
Nourseothricin ist eine in Wasser und niederen Alkoholen
lösliche Base. Handhabbare Formen sind seine Salze. Das
Molekül des Nourseothricins ist aus dem Aminozucker
Gulosamin und den Aminosäuren Streptolidin und ß-Lysin
aufgebaut. Die einzelnen Komponenten des Nourseothricin
unterscheiden sich durch die Anzahl peptidartig miteinander verknüpften ß-Lysinreste im Molekül.

Der Nourseothricin-Komplex besteht zu etwa 90 % zu annähernd gleichen Anteilen aus den beiden Hauptkomponenten F und D sowie zu etwa 10 % aus den beiden Nebenkomponenten C und E (Gräfe, U.; Bocker, H.; Reinhardt, G. und Thrum, H.: Regulative Beeinflussung der Nourseothricinbiosynthese durch o-Aminobenzoesäure in Kulturen des Streptomyces noursei JA 3890b. Zschr. Allg. Mikrobiol. 14, 659 bis 673 (1974)).

Der das Antibiotikum Nourseothricin bildende Streptomycetenstamm ist unter der Bezeichnung Streptomyces noursei ZIMET 3890b in der Stammsammlung des Zentralinstituts für Mikrobiologie und experimentelle Therapie der Akademie der Wissenschaften der DDR hinterlegt und trägt jetzt die Bezeichnung Streptomyces noursei ZIMET 43716.

Nach den aus der Literatur bekannten Verfahren (Bocker, H. und Thrum, H.: Stimulation of nourseothricin production by aminobenzoic acids. In: Herold, M. und Gabriel, Z.: Antibiotics - Advances in research, production and clinical use. London 1966; p. 584 bis 587) erfolgt die Kultivierung unter aeroben Bedingungen. Hierzu wird an Erde lyophil getrocknetes Sporenmaterial dieses Streptomyces-Stammes auf geeignete Agarnährböden aufgebracht. Nach 6- bis 10tägiger Bebrütung bei 28 bis 30 °C wird der so gewachsene versporte Myzelrasen zum Beimpfen von flüssigen sterilen Nährmedien verwendet. Die Beimpfung kann auch direkt mit einer Konserve von lyophil an Gelatine getrocknetem Submersmyzel des Nourseothricinbildenden Streptomycetenstammes erfolgen. Die flüssigen Nährmedien für die submerse Anzucht des Impfmaterials enthalten als Substrate Kohlenstoff- und Stickstoffquellen sowie anorganische Salze. Als Kohlenstoffquellen werden Glucose und/oder Glyzerin eingesetzt. Als Stickstoffquellen kommen insbesondere Sojamehl, verschiedene Aminosäuren und/oder Ammoniumsalze in Betracht. Die für die Anzucht des Impfmaterials günstigste Azidität zu Beginn der Kultivierung liegt im Bereich von pH 6,0 bis pH 6,7.

Die Bebrütung erfolgt bei Temperaturen von 28 bis 30 $^{o}$C über einen Zeitraum von 24 bis 48 Stunden.

Das so angezogene Impfmaterial dient zur Beimpfung von sterilen flüssigen Nährmedien für die Hauptkultur. Solche Nährmedien bestehen aus Kohlenstoff- und Stickstoffquellen sowie anorganischen Salzen. Als Kohlenstoffquelle werden Maisstärke und/oder Glucose eingesetzt. Als Stickstoffquelle kommen insbesondere Sojamehl, verschiedene Aminosäuren und/oder Ammoniumsalze in Betracht. Die für die Antibiotikumbildung günstigste Azidität zu Beginn der Fermentation liegt im Bereich von pH 6,0 bis pH 7,5. Die Kultivierung erfolgt bei Temperaturen von 26 bis 32 $^{o}$C, vorzugsweise bei 28 bis 30 $^{o}$C bis zu 150 Stunden.

Die submerse Kultivierung des Streptomyces noursei ZIMET JA 3890b erfolgt in bekannter Weise als Schüttelkultur oder in gerührten und belüfteten Fermentoren ohne Zudosierung von Substraten bzw. Regelung des pH-Wertes.

Bekannt ist (Bradler, G. und Thrum, H.: Nourseothricin A und B, zwei neue antibakterielle Antibiotika einer Streptomyces-noursei-Variante. Zschr. Allgem. Mikrobiol. 3, 105 bis 112 (1963)), daß die verhältnismäßig geringe Nourseothricin-Bildung auf dem ursprünglichen Fermentationsmedium durch Zusatz von Aminoaralkarbonsäuren, insbesondere von 5 bis 10 mM o-Aminobenzoesäure, zum Beginn der Hauptkultur zugesetzt, unter den bekannten technischen Bedingungen um das 5- bis 10fache gesteigert werden kann (Bocker, H. und Thrum, H.: Stimulation of nourseothricin production by aminobenzoic acids. In: Herold, M. und Gabriel Z.: Antibiotics - Advances in research, production and clinical use. London 1966, p. 584 bis 587).

Es ist aus Untersuchungen (zusammenfassend zitiert in: Gräfe, U.; Steudel, A.; Bocker, H. und Thrum, H.: Regulative influencs of o-aminobenzoic acid (OABA) on the biosynthesis of nourseothricin in cultures of Strepto-

0136256

myces noursei JA 3890b. V. Effect of OABA on cytochrom levels and amino acid transport. Zschr. Allgem. Mikrobiol. 30, 185 bis 194 (1980)) bekannt, daß o-Aminobenzoesäure und/oder andere Aminoarylkarbonsäuren spezifisch die Bildung von Cytochrom des a-Typs (Cytochrom-Oxydasen) reprimieren und dadurch indirekt sowohl den Aminosäuretransport aus dem Nährmedium in das Myzel als auch die oxydative Desaminierung der Aminosäuren in den Zellen des Nourseothricin-Bildner hemmen. Hierdurch wird einerseits eine Repression des Sekundärstoffwechsels durch ein zelluläres Überangebot an Stickstoff-Kataboliten vermieden. Andererseits wird auf diese Weise verhindert, daß als Precursoren des Nourseothricins dienende Aminosäuren des Mediums vom Antibiotikum-Bildner während dessen Wachstumsphase weitgehend aufgebraucht werden. Diese Aminosäuren sind somit dem Sekundärstoffwechsel im Verlaufe der Nourseothricin-Bildung besser verfügbar, da für die Biomassebildung bevorzugt die anorganische Stickstoffquelle (Ammonium-Stickstoff) genutzt wird.

Die fermentative Nourseothricin-Produktion unter Verwendung von Aminoarylcarbonsäuren, die bekanntermaßen zwar eine wesentliche Steigerung der Fermentationsausbeute ermöglicht, weist jedoch insbesondere hinsichtlich der Kosten, der Sterilisation und der Abwasserproblematik Nachteile auf, die einer großtechnischen Anwendung entgegenstehen.

Bekannt ist weiterhin, daß die Biosynthese der meisten Sekundärmetabolite durch überschüssiges Phosphat negativ beeinflußt wird (zusammenfassend zitiert in: Martin, J.F. und Demain, A.L.: Control of antibiotic biosynthesis. Microbiol. Rev. 44, 230 bis 251 (1980)). Deshalb werden technisch-mikrobielle Fermentationen zur Herstellung von Sekundärmetaboliten, zum Beispiel Antibiotika, bei für das Wachstum des Bildners suboptimalen Phosphatkonzentrationen durchgeführt. In der Regel werden komplexe Nährbodenbestandteile pflanzlicher und tierischer Herkunft, wie Stärke, Sojamehl, Maisquellwasser, Melasse, Fleischextrakt u. a., bei Fermentationen in technischen Maßstab zur Gewinnung von Sekundär-Metaboliten eingesetzt. Je nach Herkunft und Vorbehandlung besitzen solche komplexen Nährbodenbestandteile unterschiedliche Gehalte an Gesamtphosphat von unterschiedlicher biologischer Verfügbarkeit. Ein Teil des verfügbaren Phosphats liegt in den Fermentationsmedien als lösliches Phosphat vor. Dieser Umstand erschwert grundsätzlich die Standardisierung der Nährmedien.

Die Anfangskonzentrationen an löslichen bzw. verfügbarem Phosphat haben aber eine entscheidende Bedeutung für die fermentative Ausbeute an dem angestrebten Sekundärmetabolit, weil einerseits eine bestimmte Phosphatmenge für das Wachstum der produzierenden Mikroorganismen erforderlich ist und zum anderen zu hohe Phosphat-Anfangskonzentrationen die Sekundärmetabolit-Bildung inhibieren.

Das Patent DD 155239 beansprucht die Verwendung der Konzentration der im Kulturmedium enthaltenen Phosphationen als Regelgröße zur Stabilisierung des Kultivierungsprozesses. Das Verfahren bezieht sich jedoch nur auf die fermentative Gewinnung von Biomasse, wobei der Sollwert der Konzentration im Bereich von 20 bis 60 mg/l Phosphor einzustellen ist. Die Anwendung dieses Verfahrens auf die Regulation der Biosynthese von Sekundärmetaboliten in mikrobiellen Batch-Kulturen ist nicht bekannt.

Bei der Verbesserung mikrobieller Herstellungsverfahren für die Produktion von Sekundärmetaboliten werden das Nährmedium und der produzierende Mikroorganismus durch wechselseitiges Modifizieren so aneinander angepaßt, daß ein Kompromiß zwischen den beiden gegenläufigen regulativen Einflüssen des Phosphats zustande kommt. Dieser Weg der stufenweise Leistungssteigerung ist jedoch zeitaufwendig und kostenintensiv.

Bekannt ist ferner, daß neben der regulativen Beeinflussung durch die Zusammensetzung des Nährmediums eine Verbesserung der fermentativen Ausbeute auch über eine Steuerung des Fermentationsprozesses, vorzugsweise als Echtzeitsteuerung, zu erreichen ist (Sukatsch, D. A. und Nesemann, G.: Automatische Parametererfassung bei industriellen Fermentationen. Chemie-Technik 6, 261 (1977)).

Bei der für eine effektive Fermentationsführung erforderlichen Anwendung der Echtzeitsteuerung besteht die Schwierigkeit, auf die in der Fermentationstechnik etablierten, kontinuierlich meßbaren globalen Prozeßparameter, wie pH-Wert, $pO_2$-Wert, Dosierraten, Abgaszusammensetzung, Wärmebildung, anstelle der erst durch meist zeitaufwendige chemische Analytik nachträglich festzustellenden Substratkonzentrationen als primäre regulative Prozeßparameter ausweichen zu müssen.

Hinsichtlich der Isolierung des Nourseothricins ist schließlich bekannt, daß der Wirkstoff aus dem vom Myzel befreiten Kulturfiltrat an geeignete Kationenaustauscher adsorbiert, nachfolgend mit verdünnten Säuren eluiert und nach Neutralisation, Konzentrierung der Eluate, wiederholte Fällung des Rohprodukts im System Methanol/Azeton und Trocknung als Reinsubstanz erhalten wird. (Bradler, G. und Thrum, H.: Nourseothricin A und B, zwei neue antibakterielle Antibiotika einer Streptomyces-noursei Variante. Zschr. Allgem. Mikrobiol. 3, 105 bis 115 (1963)).

Die Erfindung hat das Ziel, den Wirkstoff Nourseothricin in hoher Raum-Zeit-Ausbeute herzustellen.

Der Erfindung liegt die Aufgabe zugrunde, ein technisch günstiges Verfahren anzugeben, das unter Vermeidung der Nachteile der bisher bekannten Lösungswege die industrielle Produktion von Nourseothricin, in Form der Salze und Adsorbate ermöglicht.

Es wurde gefunden, daß Zusätze von Hemmstoffen der Atmungskette, beispielweise Natriumazid bzw. andere Alkaliazide, Brenzkatechin sowie Amytal (= Ethylisoamylbarbitursäure) und/oder Hemmstoffe des Aminosäuretransports in der lebenden Zelle, beispielsweise ß-Alanin oder wasserlösliche Zinksalze, der Hauptkultur zum Zeitpunkt der Beimpfung zugesetzt, eine fördernde Wirkung auf die Biosynthese von Nourseothricin ausüben, ohne die chemischen und biologischen Eigenschaften des Fermentationsprozesses ändernd zu beeinflussen.

Ein Zusatz von Inhibitoren der Atmungskette und/oder des Aminosäuretransports kann entfallen, wenn Stämme hoher amylolytischer Aktivität verwendet und polymere Kohlenstoffquellen, vor allem Maisstärke, eingesetzt werden. Überraschenderweise wurde gefunden, daß die metabolische Aktivität der Nourseothricin-bildenden Kulturen (Respiration, Säuerungsrate, Glucose- und Ammoniumaufnahme, Antibiotikumbildung u. a.) primär durch die spezifische Verfügbarkeit des Phosphats im Verlaufe der Fermentation und nicht allein vom Angebot an Kohlenstoff- und Stickstoffquellen bestimmt wird. Dabei ist es von besonderer Bedeutung, daß generell die Anfangskonzentrationen an löslichem und auch an biologisch verfügbarem Phosphat im sterilisierten Nährmedium durch Führung der thermischen Sterilisation auf Basis eines Sterilisationsfunktionals, des Gehalts an Sulfation und an anorganischen Salzen bzw. Metallionen, die mit Phosphat- bzw. Sulfationen schwerlösliche Sedimente ergeben, erniedrigt werden. Dabei hat sich gezeigt, daß für die Absenkung dieser Anfangskonzentrationen im Fall der Anwesenheit einer hinreichenden Menge von Sulfationen die Verschiebung der Azidität des schwach sauren Nährmediums zu alkalischen pH-Werten im Verlaufe der Sterilisation durch Ausfällen von Calciumsulfat und die damit verbundene Entfernung der sauren Sulfationen aus dem Medium verantwortlich ist. Danach wird die Anfangskonzentration an löslichem bzw. biologisch verfügbarem Phosphat auch durch Einstellung eines alkalischen pH-Wertes vor der Sterilisation erniedrigt. Die Erhöhung des pH-Wertes bewirkt, daß Bedingungen für die Ausfällung des Phos-

phats als schwerlösliche Sedimente mit bestimmten, im Nährmedium vorhandenen Metallionen gegeben sind. Die Ausfällung der Phosphat-haltigen Sedimente wird durch die gezielte Zugabe solchen Metallionen, beispielsweise von Zink(II) und/oder Eisen(III), Aluminium(III), Magnesium(II), Calcium(II), Mangan(II) u. a., gefördert. Besonders günstig für eine Phosphat-Ausfällung ist die bei den meisten technischen Fermentationen verwendete Sterilisationsmethode mit direkt einwirkendem gespannten Wasserdampf sowie die Anwesenheit von Calciumcarbonat, das zu pH-Pufferung der Fermentation eingesetzt wird.

Der Phosphatgehalt des Mediums kann generell auch durch Verringerung des Anteils der Phosphat-haltigen komplexen Einsatzstoffe erniedrigt werden. Dadurch wird das Verfahren kostengünstiger.

Unter den geschilderten Voraussetzungen wird die Phosphat-Anfangskonzentration an gelöstem bzw. biologisch verfügbarem Phosphat so erniedrigt, daß die für den Nourseothricin-produzierenden Mikroorganismus spezifischen oberen Toleranzwerte des Phosphats in jedem Fall unterschritten werden. Diese geringen Phosphat-Konzentrationen bieten nun die Möglichkeit, durch eine definierte Phosphat-Zuführung nach der Sterilisation ein in Bezug auf das Phosphat weitgehend standardisiertes Medium zu erhalten. Darüber hinaus kann über das Zugaberegime des Phosphats der Phosphat-Stoffwechsel der Mikroorganismen so gesteuert werden, daß eine hohe Produktausbeute erhalten wird. Die Zuführung des Phosphats kann in Form einer Lösung oder eines Feststoffes oder einer Suspension durchgeführt werden. Das Phosphat kann entweder als freie Phosphationen oder als chemisch oder physikalisch gebundenes Phosphat vorliegen. Je nach Fermentationsregime erfolgt die Zuführung von Phosphat bzw. phosphathaltigen Substanzen nach der Sterilisation oder während der Fermentation durch eine einmalige und/oder mehrmalige und/oder eine kontinuierliche Zugabe. Die kontinuierliche Zugabe kann mit unter-

schiedlichen Raten während der Fermentation durchgeführt werden.

Diese Zugabe erfolgt additiv zu dem in der Kulturlösung aus den komplexen Phosphatquellen, beispielsweise Sojamehl, Erdnußmehl, Kartoffelstärke u. a., durch hydrolytischen oder enzymatischen Aufschluß bzw. durch reversible Freisetzung aus anorganischen Sedimenten freiwerdenden Phosphat. Der hydrolytische Aufschluß der vorgenannten organischen Substrate wird durch Sterilisation bei alkalischer Reaktion gefördert. Durch eine Erhöhung der Wasserstoffionenkonzentration im Fermentationsverlauf innerhalb des physiologischen Bereichs wird durch eine bessere Freisetzung von Phosphat aus anorganischen Sedimenten eine Stimulation der metabolischen Aktivitäten erreicht.

Als Randbedingung ist zu sichern, daß keine für die Produktbildung ungünstige Limitation von Substraten oder durch Umweltbedingungen im Zusammenhang mit der Phosphatzuführung und daraus resultierendem Wachstum bzw. gesteigerten Stoffwechselaktivitäten entstehen.

Nach alkalischer Sterilisationsführung im pH-Wert-Bereich von 7,4 bis 7,8 mit Beendigung der Sterilisation und Erreichen eines pH-Wert-Bereiches von 7,2 bis 8,8 wurde weiterhin gefunden, daß bei Steuerung des Fermentationsprozesses durch Einstellung und Aufrechterhaltung eines Verhältnisses von Glucose zu anorganischem Stickstoff von 5 bis 15 zu 0,015 bis 0,2 (g/l Glucose : g/l anorganischem Stickstoff) mittels Dosierung von Glucose und Ammoniumsalzen bzw. Ammoniumhydroxid im Azidititätsbereich von pH 5,0 bis 6,5 und einem Sauerstoffpartialdruck ($pO_2$) von 30 bis 80 %, vorzugsweise 40 bis 60 %, die Produktivität der Nourseothricinbildung über einen längeren Zeitraum günstig beeinflußt wird.

Überraschenderweise wurde gefunden, daß die Wahl des passenden Sollwertintervalls für die Regelung des pH-Wertes der Kulturlösung im Bereich physiologisch-regulativ effektiver pH-Werte bei Zudosierung einer geeignet konzentrierten Ammoniumhydroxid-Lösung zur Gewährleistung der
unteren pH-Grenze bewirkt, daß der Zeitverlauf der Ammoniumhydroxid-Dosierrate den Zeitverlauf der Verbrauchsrate des physiologisch wirksamen Phosphats abbildet. Der
Abbildungsmaßstab hängt von der Festlegung der unteren
Regelgrenze des pH-Werts ab. Der pH-Verlauf in den ersten
Fermentationsstunden und damit das Einsetzen der Ammoniumhydroxid-Dosierrate sind durch die Pufferung des Startnährmediums bestimmt.

Damit steht ein kontinuierliches Signal guter Empfindlichkeit für die Steuerung der direkten und/oder indirekten Phosphatdosierung und damit für die Steuerung der
metabolischen Aktivität der Fermentationskultur zur Verfügung.

Bezüglich der direkten Phosphatdosierung in Form der Zugabe phosphathaltiger Substanzen aus einem extremen Reservoir und der indirekten Phosphatdosierung als Bereitstellung von Phosphatverbindungen durch enzymatischen
oder hydrolytischen Aufschluß komplexer Substrate wurde
gefunden, daß der Betrag der Ammoniumhydroxid-Dosierrate
Übergänge zwischen direkter und indirekter Phosphatdosierung durch signifikantes Anwachsen bzw. Abfallen anzeigt. Die Größe der Änderung wird durch den Phosphat-
nachfluß bestimmt.

Außerdem wurde gefunden, daß die aktuelle Ammoniumhydroxid-Dosierrate als Steuergröße für die Dosierung von
Kohlenstoffquellen sowie weiteren Stickstoffquellen und
Substanzen mit Effektorwirkung dient und unter Beachtung
der spezifischen Sauerstoffübergangsrate des betreffenden Fermentors die direkte Phosphatdosierung so abzuändern ist, daß die Fermentation mit günstiger Produktbildungsrate verläuft. Auf diese Weise gelingt unter den reaktortechnisch gegebenen Sauerstoff-Eintragungsbedingungen bei festgelegter Zuluftmenge die Realisierung eines hohen Produktendwertes, die effektive Verwertung der

- 11 -

0136256

Substrats im angestrebten Fermentationszeitraum und die Beschränkung der Biomassekonzentration auf das aufarbeitungstechnisch vertretbare Maß.

Weiterhin wurde gefunden, daß der Zeitvorlauf der metabolischen Aktivität durch die Reaktionswärme und/oder die Respirationsrate abgebildet wird. Aus diesem Grunde sind die aktuelle Reaktionswärme und die Respiration ebenfalls Steuergrößen für die Dosierung von Phosphat bzw. anderen Substanzen.

Für das Herstellungsverfahren sind Nourseothricin-bildende Streptomycetenstämme der Gattung Streptomyces, insbesondere der Art Streptomyces noursei, geeignet, die durch die nachstehenden Kriterien charakterisiert werden:

- Empfindlichkeit der Nourseothricin-Bildung gegen hohe Konzentrationen an löslichem Phosphat im Nährmedium der Hauptkultur

- Fähigkeit zum Aufschluß komplexer phosphathaltiger Kohlenstoff- und Stickstoffquellen

- Amylolytische Aktivität zum Aufschluß der polymeren Kohlenstoffquellen.

Die konkrete Durchführung des beschriebenen Verfahrens richtet sich danach, wie der jeweils eingesetzte Streptomycetenstamm hinsichtlich der 3 oben genannten Kriterien einzuordnen ist.

So wird im aufgezeigten Verfahrensrahmen in einem Grenzfall bei hoher Phosphatempfindlichkeit des Produzenten eine hohe Nourseothricinbildung nur dann möglich, wenn die Anfangskonzentration an löslichem Phosphat im Nährmedium der Hauptkultur im Bereich unter 10 mg/l Phosphat-Phosphor mittels der weiter vorn beschriebenen Maßnahmen, wie Hitzesterilisation im alkalischen Milieu und/oder Zusatz von Phosphat-fällenden Substanzen abgesenkt wurde.

Beim Einsatz von Nourseothricin-bildenden Streptomycetenstämmen mit hoher Fähigkeit zum Aufschluß komplexer phosphathaltiger Substrate, deren ausreichendes Vorliegen im

0136256

Nährmedium vorausgesetzt ist, kann die Dosierung an Phosphat während der Fermentation bis zum Wert 0 verringert werden.

Beim Einsatz von Nourseothricin-bildenden Streptomyceten-stämmen mit hoher Phosphatresistenz kann der Zusatz von Phosphat-fällenden Substanzen zum Nährmedium für die Hauptkultur bis zum Wert 0 verringert werden. Es wurde gefunden, daß solche Streptomycetenstämme der Art Streptomyces noursei gleichzeitig eine hohe amylolytische Aktivität besitzen.

Diese Ausführungen zeigen, daß physiologisch unterschiedliche Streptomycetenstämme für das Herstellungsverfahren eingesetzt werden. Daher ist auch kein besonders spezifizierter Streptomycetenstamm kennzeichnendes Merkmal der Erfindung.

Bezüglich der Gewinnung des Wirkstoffs aus der Kulturlösung wurde überraschenderweise gefunden, daß Nourseothricin in Form eines mycelhaltigen Adsorbats isoliert werden kann. Hierzu wird nach Abbruch der Fermentation die Kulturlösung durch Zusatz von verdünnter Schwefelsäure auf eine schwach saure Reaktion, insbesondere pH 6,0 bis 6,2 eingestellt. Anschließend wird eine schwach saure, wässerige Suspension eines physiologisch unbedenklichen Adsorbens, beispielsweise natrifizierter Bentonit, der angesäuerten Kulturlösung gut untergerührt. Hierbei ist zu beachten, daß die Azidität der Reaktionslösung sich im Bereich von pH 5,5 bis 6,5 gehalten wird. Der sich absetzende Nourseothricin-haltige Feststoff wird durch Filtration oder Zentrifugation abgetrennt und im Warmluftstrom bei einer Produkttemperatur von höchstens 70° getrocknet. Das so hergestellte mycelhaltige Adsorbat enthält 1 bis 10 %, überwiegend 4 bis 7 % Wirkstoff, berechnet als Nourseothricin-Base.

Weiterhin wurde überraschenderweise gefunden, daß bestimmte Nourseothricin-Salze in 90 bis 95%igem wäßrigen Methanol schwerlöslich sind und unter den nachfolgend beschriebenen Verfahrensbedingungen isoliert und gereinigt werden können. Hierzu werden Adsorbate des Nourseothricins mit schwach sauren Kationenaustauschern mit solchen verdünnten mehrbasischen Säuren, die mit Nourseothricin-Base methanolunlösliche Salze bilden, eluiert. Anschließend werden die in den Eluaten als Begleitstoffe vorhandenen mehrwertigen anorganischen Kationen als im Wasser schwer lösliche Salze ausgefällt. Danach werden die einwertigen anorganischen Kationen durch Behandlung der Eluate mit einem hochvernetzten Kationenaustauscher vom Sulfonsäuretyp in H-Form entfernt und die freiwerdenden Säuren mit einem Anionenaustauscher in OH-Form neutralisiert. Nach dem Konzentrieren der Eluate im Vakuum und Entfernen der Verunreinigungen durch Adsorption an Aktivkohle erfolgt schließlich die Gewinnung der reinen Salze des Nourseothricins durch Methanolfällung oder schonende Trocknung.

Nourseothricin-Sulfat ist ein amorphes, weißes, im Wasser leicht lösliches, in Methanol und den meisten organischen Lösungsmitteln schwer lösliches Pulver. Die Elementarzusammensetzung (C = 32,46, 32,31; H = 6,65, 6,22; N = 15,73, 16,00; S = 7,74, 7,60) entspricht weitgehend der chemischen Zusammensetzung eines 1 : 1 Gemisches von Streptothricin D-($C_{31}H_{58}N_{12}O_{10}$ · 2,5$H_2SO_4$ · $H_2O$) und Streptothricin F-Sulfat ($C_{19}H_{34}N_8O_8$ · 1,5$H_2SO_4$ · $H_2O$).

Nourseothricin-Oxalat ist ein amorphes, weißes, in Wasser leicht lösliches, in Alkoholen und anderen organischen Lösungsmitteln schwer lösliches Pulver. Die Elementarzusammensetzung (40,31, 40,44; H=6,42, 6,21; N=16,21, 16,49) sowie der Oxalsäuregehalt (20,12, 20,28) entsprechen in etwa der Zusammensetzung eines 1 : 1 Gemisches von Streptothricin D-($C_{31}H_{58}N_{12}O_{10}$ · 2,5$C_2H_2O_4$ · 1-3$H_2O$) und Streptothricin F-Oxalat ($C_{19}H_{34}N_8O_8$ · 1,5 $C_2H_2O_4$ · 1-3$H_2O$).

Nourseothricin-Phosphat ist eine amorphe, weiße, in Wasser leicht lösliche, in Alkoholen und anderen organischen Lösungsmitteln schwer lösliche Substanz. Die Elementarzusammensetzung (31,59, 31,85; H 6,25, 6,63; N 14,95 15,18; P 10,08, 10,36) entspricht der folgenden Zusammensetzung eines 1 : 1 Gemisches von Streptothricin-D-($C_{31}$ $H_{58}N_{12}O_{10}$ · 4 $H_3PO_4$ · 0-1 $H_2O$) und Streptothricin-F-Phosphat ($C_{19}H_{34}N_8O_8$ · 2 $H_3PO_4$ · 0-1 $H_2O$).

Die relative antimikrobielle Antivität der so erhaltenen Salze kann mikrobiologisch mit Hilfe des Lochplatten-Diffusionstestes unter Verwendung von Bacillus rubtilis ATCC 6633 als Testorganismus ermittelt werden. Als Standard wird ein gereinigtes Nourseothricin-Sulfat verwendet, das in seiner Zusammensetzung einem 1 : 1 Gemisch von Streptothricin D-($C_{31}H_{58}N_{12}O_{10}$ · 2,5 $H_2SO_4$ · $H_2O$) und Streptothricin F-Sulfat ($C_{19}H_{34}N_8O_8$ · 1,5 $H_2O$) ent- und 702 ug Base/mg enthält. Die Bedingungen für die mikrobiologische Wertbestimmung sind so zu wählen, daß 0,05 ml einer 10 ug Nourseothricin-Base pro ml enthaltenden Prüflösung bei einem Stanzlochdurchmesser von 9 mm eine Hemmzone von 19 ± 1 mm Durchmesser ergeben.

Ausführungsbeispiele

Beispiel 1

Eine lyophil an Erde getrocknete Sporenkonserve des Stammes Streptomyces noursei ZIMET 43716 wird auf ein geeignetes Agarmedium, beispielsweise Emerson-Agar, ausgestreut. Nach etwa 7tägiger Bebrütung bei 30 °C werden etwa 2 cm$^2$ große Stücken des Mycelrasens herausgeschnitten und damit jeweils ein Kulturansatz der 1. Vorkultur beimpft.

Das Medium dieser Vorkultur hat folgende Zusammensetzung pro Liter: 40,0 g Glucose; 15,0 g Sojaextraktionsschrot; 0,3 g $KH_2PO_4$; 5,0 g NaCl; 3,0 g $CaCO_3$; Leitungswasser ad 1000 ml; pH 6,5 bis 6,9 (vor Sterilisation).

Dieses Medium ist in 500 ml fassende, mit Wattestopfen verschlossene Steilbrustflaschen in Mengen von jeweils 50 ml abgefüllt. Die Sterilisation erfolgt durch 35 Minuten langes Erhitzen auf 121 °C im Autoklaven.

Die beimpften Ansätze werden 48 Stunden als Schüttelkulturen bei 29 °C bebrütet. Mit jeweils 3 ml der so erhaltenen Vorkulturen werden die Kulturenansätze der Hauptkultur beimpft.

Für die Hauptkultur werden pro 1 Leitungswasser folgende Grundmedien eingesetzt:

Medium Bo-30    29,0 g Glucose; 13,0 Sojaextraktionsschrot; 5,0 g NaCl; 3,0 g $CaCO_3$; Leitungswasser ad 1000; pH 6,5 (vor Sterilisation)

Medium Bo-31    30,0 g Maisstärke; 3,0 g Glucose; 7,0 g Sojaextraktionsschrot; 5,0 g $NH_4NO_3$; 2,0 g $MgSO_4$; 5,0 g NaCl; 3,0 g $CaCO_3$; Leitungswasser ad 1000; pH 6,0 (vor Sterilisation).

Die Nährmedien der Hauptkultur werden in Mengen zu jeweils 80 ml in 500 ml fassende, mit Wattestopfen verschlossene Steilbrustflaschen abgefüllt und 30 Minuten bei 121 °C im Autoklaven sterilisiert.

Entsprechend des Versuchsplanes werden die so behandelten Ansätze der Hauptkultur zum Zeitpunkt der Beimpfung mit sterilfiltrierten, wässerigen, neutralen Lösungen der betreffenden in den Tabellen 1 und 2 aufgeführten Substanzen (maximal 3,0 ml Zusatz/80 ml Medium) verwendet.

Die während der 72 bis 120stündigen Bebrütung der Hauptkulturen auf Schütteltischen (180 U/min) bei 26 °C mit Hilfe des Lochplattendiffusionstestes unter Verwendung von Bacillus subtilis ATCC 6633 als Testkeim ermittelten maximalen Nourseothricin-Konzentrationen sind in den Tabellen 1 und 2 angegeben. Die weitere Aufarbeitung erfolgt nach Beispielen 8 bis 13.

Beispiel 2

Eine lyophil an Erde getrocknete Sporenkonserve der Selektante NG 13-14 des Nourseothricin-bildenden Stammes ZIMET 43716 wird auf ein geeignetes Agarmedium, beispielsweise Emerson-Agar, ausgestreut. Nach etwa siebentägiger Bebrütung bei 30 °C werden etwa 2 cm$^2$ große Stücken des Myzelrasens herausgeschnitten und damit jeweils ein Kulturansatz der Vorzucht beimpft. Das Vorzuchtmedium enthält je Liter Leitungswasser folgende Einsatzstoffe: 40 g Glucose; 15 g Sojaextraktionsschrot; 0,3 g $KH_2PO_4$; 5 g NaCl und 3 g $CaCO_3$. Die Azidität wird vor der Sterilisation auf den Wert pH 6,5 eingestellt. Dieses Medium wird in 500 ml fassense, mit Wattestopfen verschlossenen Steilbrustflaschen in Mengen zu jeweils 50 ml abgefüllt. Die Sterilisation erfolgt durch 35minütiges Erhitzen auf 121 °C im Autoklaven.

Die beimpften Vorzuchtansätze werden 48 Stunden als Schüttelkulturen bei 29 °C bebrütet. Mit 150 ml der so erhaltenen Vorzuchten wird der 2500 ml beinhaltende Kulturansatz der Hauptkultur im Laborfermentor beimpft.

Für die Hauptkultur wird das Medium Bo-34 verwendet, wel-

ches im Liter Leitungswasser folgende Einsatzstoffe enthält: 32 g Kartoffelstärke; 29 g Glucose; 11 g Sojaextraktionsschrot; 11 g $(NH_4)_2SO_4$; 2 g $MgSO_4 \cdot 7H_2O$; 1 g NaCl; 6 g $CaCO_3$ und 0,5 g $ZnSO_4 \cdot 7H_2O$. Auf 2500 ml Nährmedium werden als Entschäumer auf Silikonbasis 1 ml Antaphron zugegeben. Vor der Sterilisation hat die Azidität den Wert pH 6,0. Nach der Steilisation des mit 2500 ml gefüllten Fermenter-Kulturgefäßes bei 121 °C im Autoklaven und Wiederabkühlen auf 30 °C wird die Start-Azidität durch aseptischen Zusatz von 10%iger steriler Schwefelsäure auf den Wert pH 6,8 eingestellt. Die beimpften Fermentationsansätze werden 168 Stunden bei einer Temperatur von 30 °C und einer Belüftungsrate von 2500 ml Luft pro Minute unter Rühren von 800 Umdrehungen $\cdot$ $min^{-1}$ (U/min) bebrütet. Ab 1,5 Stunden nach Beimpfung der Hauptkultur wird eine Lösung von 4 g $KH_2PO_4$ pro Liter destilliertem Wasser über einen Zeitraum von 5 Stunden mit einer Rate von 1,8 ml/Stunde mittels einer Peristaltikpumpe kontinuierlich zudosiert; anschließend erfolgt diese Zugabe über einen Zeitraum von 21 Stunden mit einer Dosierrate von 3,5 ml/Stunde. Während der Fermentation wird unter aseptischen Bedingungen periodisch Proben der Kulturlösung entnommen und in diesen die Gehalte an Nourseothricin-Base mittels des Lochplattendiffusionstests unter Verwendung von Bacillus subtilis ATCC 6633 als Testkeim, an Glucose, an Ammoniumstickstoff und an Feststoffen bestimmt. Die Parameter $pO_2$, pH und $CO_2$-Gehalt in der Abluft werden während des Prozeßverlaufes durch registrierende Messung verfolgt. Das Absinken der Azidität der Kulturlösung unter den Wert pH 5,8 wird durch Zudosierung von steriler 5%iger Natronlauge verhindert.

Bei Absinken der Glucose-Konzentration auf 5 g/l oder des Ammoniumstickstoffs auf 0,5 g/l, erkennbar an einer Erhöhung der pH-Werte und der $pO_2$-Werte sowie Erniedrigung des $CO_2$-Gehalts in der Abluft, werden 25 g Glucose bzw. 10 g $(NH_4)_2SO_4$ als konzentrierte, sterile Lösungen diskontinuierlich zugegeben.

Eine weitere Fermentation wird ohne Zudosierung von $KH_2PO_4$, Glucose und $(NH_4)_2SO_4$ unter ansonsten gleichen Bedingungen durchgeführt. Bei Abbruch der Fermentation weist die Kulturlösung noch deutlich nachweisbare Mengen an freier Glucose und freiem Ammonium auf.

Die in den beiden unterschiedlichen Fermentationen im Kulturfiltrat bestimmten Konzentrationen an Nourseothricin-Base wird in der Tabelle 3 wiedergegeben.

## Beispiel 3

Eine lyophil an Glucose-Gelatine getrocknete Myzelkonserve (Bruttogewicht des Konserveinhaltes etwa 300 mg) des im Beispiel 2 genannten Nourseothricin-bildenden Streptomyces-Stammes wird mit 3 ml einer 0,9%igen NaCl-Lösung aufgeschwemmt. Von dieser Suspension dienen 0,5 ml als Inokulum von 400 ml Vorzucht der ersten Stufe. Das Vorzucht-Medium hat die gleiche Zusammensetzung und Azidität wie im Beispiel 2 angegeben. Dieses Medium wird in 2500 ml fassende, mit Wattestopfen verschlossenen Steilbrustflaschen in Mengen von je 400 ml abgefüllt. Die Sterilisation erfolgt durch 35 Minuten dauernde Erhitzung auf 121 °C im Autoklaven.

Die auf Raumtemperatur rückgekühlten und beimpften Ansätze werden 48 Stunden als Schüttelkulturen (180 U/min) bei 29 °C bebrütet. Mit 1200 ml der so erhaltenen Kulturlösung der ersten Vorzucht wird als zweite Stufe der Vorzucht ein mit 150 Liter hitzesterilisiertem Medium (60 Minuten bei 121 °C) der gleichen Zusammensetzung wie in der ersten Vorkulturstufe gefüllter, mit Vorrichtungen zur Sterilbelüftung und einem Rührwerk ausgestatteter Edelstahlfermenter (250 Liter Bruttovolumen) beimpft. Die Kultivierung erfolgt bei 27 bis 29 °C, einer Geschwindigkeit das Rührwerks von 240 U/min und einer Belüftungsrate von 1 Liter Luft pro Liter Kulturlösung in der Minute.

0136256

Für die Hauptkultur dienen mit Vorrichtungen zur Sterilbelüftung und einem zentralgelagerten Rührwerk versehene Edelstahlfermenter (710 Liter Bruttovolumen), die mit je 500 Liter Nährmedium Bo-34 (Rezeptur siehe Beispiel 2) gefüllt wird. Als Entschäumer werden 0,5 g/l Antaphron zugesetzt. Die Azidität wird vor der Sterilisation auf den pH-Bereich 6,7 bis 7,0 eingestellt. Die Sterilisa-tion des Nährmediums (ohne Glucose-Anteil) erfolgt in situ durch Vorwärmung mit Manteldampf auf etwa 70 $^{o}$C und anschließender Erhitzung mittels Direktdampf auf 121 $^{o}$C für 15 Minuten. Nach der Abkühlung auf etwa 30 $^{o}$C wird die in Form einer 50%igen wäßrigen Lösung durch 30minütiges Erhitzen auf 121 $^{o}$C getrennt sterilisierte Glucose unter aseptischen Bedingungen zugesetzt. Vor der Beimpfung erfolgt die Einstellung der Start-Azidität auf Werte zwischen pH 6,9 bis 7,1 mittels steriler 10%iger Schwefelsäure.

Die mit jeweils 5 % Inokulum aus der zweiten Vorzuchtstufe beimpften Fermenter-Ansätze der Hauptkultur werden 120 Stunden bei Temperaturen zwischen 28 bis 30 $^{o}$C, einer Belüftungsrate von 500 Liter Luft pro Minute (Druck 0,14 bis 0,16 MPa) unter Rühren (320 U/min) kultiviert.

Zu einer der beiden Fermenter-Ansätze wird ab der 2. Stunde nach der Beimpfung 87 g $KH_2PO_4$ als wäßrige Lösung mittels Peristaltikpumpe mit konstanter Rate dosiert.

Bei Absinken der Azidität unter pH 6,3 wird die Rückstellung von diesem pH-Wert mittels steriler 25%iger wäßriger Ammoniumhydroxid-Lösung vorgenommen.

Während der Fermentation werden unter aseptischen Bedingungen periodisch Proben der Kulturlösung entnommen und in diesen die Konzentrationen an Nourseothricin-Base mittels des Lochplattendiffusionstestes unter Verwendung von Bacillus subtilis ATCC 6633 als Testkeim, an Glucose, an Ammoniumstickstoff und an Feststoffen bestimmt. Die Parameter pH und $CO_2$-Gehalt in der Abluft werden während des Prozeßverlaufes durch registrierende Messung ver-folgt.

Beim Absinken der Glucose-Konzentration unter 5 g/l oder des Ammoniumstickstoffs unter 0,5 g/l, erkennbar an einer Erhöhung der gemessenen pH-Werte und einer Erniedrigung des $CO_2$-Gehalts in der Abluft, werden 5000 g Glucose bzw. 1800 g Ammoniumsulfat als konzentrierte, sterile wäßrige Lösungen diskontinuierlich zugegeben.

Der andere Fermenter-Ansatz erhält keine Zudosierung an Phosphat, Glucose bzw. Ammoniumsulfat. Eine pH-Regulierung erfolgt nicht; die kontinuierlich gemessenen Aziditäts-Werte bewegen sich in den Bereich von pH 6,4 bis pH 7,2. Bei Abbruch der Fermentation enthält die Kulturlösung noch Mengen über 5 g/l Glucose und 0,5 g/l Ammoniumstickstoff.

Die in den beiden unterschiedlichen Fermentationen im Kulturfiltrat bestimmten Konzentrationen an Nourseothricin-Base werden in der Tabelle 4 wiedergegeben.

Beispiel 4

Die Suspension einer Myzellyophilkonserve in physiologischer Kochsalzlösung eines Leistungsstammes von Streptomyces noursei dient als Inoculum für die 1. Submerspassage.
Das Nährmedium enthält im Liter Leitungswasser: 15 g Glucose; 15 g Sojaextraktionsschrot; 0,3 g $KH_2PO_4$; 5,0 g NaCl; 1,0 g $CaCO_3$; pH 6,5 bis 6,9 (Abfüllung zu je 50 ml in 500 ml Schüttelkolben).

Die Sterilisation des Mediums erfolgt bei 120 °C 35 Minuten. Nach 48stündiger Kultivierung bei 28 °C auf einer Rotationsschüttelmaschine wird die 2. Submerspassage (250 ml Abfüllung, 2000 ml Schüttelkolben) im Verhältnis von 1 Teil Inoculum zu 25 Teilen Nährmedium über 24 Stunden auf der Rotationsschüttelmaschine kultiviert.
600 ml der 2. Submerspassage werden für die Beimpfung von 800 l Impffermentermedium verwendet.
Das Nährmedium enthält im Liter: 15 g Glucose; 15 g Sojaextraktionsschrot; 0,3 g $KH_2PO_4$; 5,0 g NaCl; 1,0 g

$CaCO_3$; 5 g Antaphron; 37,5 g Sonnenblumenöl.

Der pH-Wert wird vor der Sterilisation auf 7,2 bis 7,4 eingestellt, er beträgt nach Beendigung der Sterilisation 6,8 bis 7,0. Die Sterilisation erfolgt bei 120 $^o$C 60 Minuten. Nach ca. 30 Stunden Kultivierung bei 28 $^o$C werden ca. 60 mg/l lösliches Phosphat verbraucht und dabei 12 bis 15 g/l Biomasse gebildet. Die Inoculummenge von 800 l ist ausreichend für die Beimpfung von ca. 18 $m^3$ Produktbildungsmedium der Hauptkultur.

Das Nährmedium des Produktionsfermentors enthält im Liter: 32,0 g Kartoffelstärke; Sojaextraktionsschrot 15,0 g 1,0 g NaCl; 6,0 $CaCO_3$; 0,5 g $ZnSO_4 \cdot 7H_2O$; 2,0g$MgSO_4 \cdot 7H_2O$; 3,0 g Sonnenblumenöl; 0,3 g Antaphron; 29,0g Glucose; 6,0 g $(NH_4)_2SO_4$.

Die Komponenten Glucose und Ammonsulfat werden nach separater Sterilisation (120 $^o$C, 30 Minuten) dem Nährmedium zugeführt.

Die Sterilisation des Mediums erfolgt bei 115 $^o$C 60 Minuten, wobei vor Beginn ein pH-Wert von 7,5 bis 7,8 eingestellt wird, der nach Beendigung der Sterilisation Werte zwischen 7,2 bis 8,2 erreicht.

Das komplettierte Medium erreicht pH-Werte zwischen 7,0 bis 7,4. Die Fermentation erfolgt bei 28 $^o$C mit einem Belüftungsverhältnis von 0,3 VVM (Volumen Luft pro Volumen Kulturlösung in einer Minute) bei 0,02 MPa und einem Sauerstoffpartialdruck von 60 %.

Bei Unterschreitung der Grenzwerte für Glucose von 15 g/l und Ammoniumstickstoff 0,2 g/l werden diese Komponenten als 50%ige sterile Lösungen zudosiert, bis die Kulturlösung einen pH-Wert von 5,2 bei einem Ammoniumstickstoffwert über 300 mg/l erreicht.

Die weitere Ammoniumstickstoffzugabe erfolgt über die Dosierung von Ammoniakwasser bei konstantem pH-Wert von 5,5. Nach einer Fermentationszeit von 130 Stunden wird eine biologische Aktivität von 8000 bis 11000 ug Nourseothricin/ml Kulturlösung gebildet.

Beispiel 5

Zur Impfmaterialanzucht werden 2,5 1-Steilbrustflaschen, die je 400 ml sterilisiertes Nährmedium, das im Liter: 40 g Glucose; 15 g Sojaextraktionsschrot; 0,3 g $KH_2PO_4$; 5 g NaCl; 3 g $CaCO_3$; Leitungswasser ad 1000 enthalten, mit 0,5 ml einer Myzelkonserven-Aufschwemmung einer Selektante von Streptomyces noursei ZIMET 43 716 beimpft. Die Kultivierung erfolgt bei 29 $^{o}$C über 48 Stunden auf einem Rundschwingschütteltisch.

Mit je 800 ml der so erhaltenen Kulturlösung der ersten Vorzuchtstufe werden als zweite Vorzuchtstufe mit 180 1 dampfsterilisiertem Medium (45 Minuten bei 120 $^{o}$C) der gleichen Zusammensetzung wie in der ersten Vorzuchtstufe gefüllte Edelstahlfermentoren (Bruttovolumen 2401) beimpft. Die Kultivierung erfolgt über 40 Stunden bei 28 bis 29 $^{o}$C, einer Rührerdrehzahl von 240 U/min und einer Belüftungsrate von 1,0 VVM.

Für die Hauptkultur werden Edelstahlfermentoren (Bruttovolumen 4200 1) eingesetzt, die mit 2500 1 Nährlösung folgender Grundzusammensetzung gefüllt sind: 62,5 g Maisstärke; 2,5 g Glucose; 16 g Sojaextraktionsschrot; 11 g$(NH_4)_2SO_4$; 2 g $MgSO_4$ • $7H_2O$; 1 g NaCl; 6 g $CaCO_3$; 0,5 g Polypropylenglykol; Leitungswasser ad 1000. Zu diesen Grundansätzen kommen in einem Fall je Liter Leitungswasser 0,5 g $ZnSO_4$ • $7H_2O$ in anderem Falle 0,1 g $Fe_2(SO_4)_3$ • $7H_2O$. Die Azidität der Nährlösung wird vor der Sterilisation auf den pH-Wert 6,2 eingestellt. Die Sterilisation (ohne Glucoseanteil) erfolgt durch Erhitzung mittels Direktdampf auf 120 $^{o}$C für 15 Minuten. Nach Abkühlung auf 29 $^{o}$C wird die in Form einer 50%igen wäßrigen Lösung getrennt sterilisierte Glucose aseptisch zugesetzt. Vor der Beimpfung erfolgt die Einstellung der Start-Azidität der Nährlösung mit Schwefelsäure auf den pH-Wert 6,8 ± 0,1. Die Fermentationsansätze werden mit 4 % Inoculum aus der zweiten Vorzuchtstufe beimpft. Die Kultivierung erfolgt über 140 Stunden bei 29 $^{o}$C, einer Belüftungsrate von

- 23 - . 0136256

0,5 VVM (0. bis 20. Stunde) bzw. 1,0 VVM (20. bis 140. Stunde) bei 0,03 MPa Überdruck unter Rühren mit 180 U/min. Die Azidität der Kulturlösung wird nach dem Absinken auf einen pH-Wert von 6,0 $\pm$ 0,1 mit 25%iger Ammoniumhydroxydlösung konstant geregelt.

In den Kulturlösungen werden im biologischen Test folgende Nourseothricinkonzentrationen bestimmt:

| Fermentationsstunde (h) | Nourseothricinkonzentration ($\mu$g/ml) | |
|---|---|---|
| | $ZnSO_4 \cdot 7H_2O$ | $Fe_2(SO_4)_3$ |
| 92. | 4,9 | 5,1 |
| 116. | 7,1 | 6,8 |
| 140. | 8,3 | 8,0 |

Beispiel 6

Zur Impfmaterialanzucht werden in der ersten Stufe 2,5 l Steilbrustflaschen, die je 400 ml sterilisiertes Nährmedium (15 g Erdnußextraktionsschrot; 10 g Maisquellwasser (100 % Trockensubstanz); 40 g Maltose; 5 g $(NH_4)_2SO_4$; 6 g $CaCO_3$; Leitungswasser at 1000) enthalten, mit je 0,5 ml einer Myzelkonserven-Aufschwemmung einer Selektante von Streptomyces noursei ZIMET 43 716 beimpft. Die Kultivierung erfolgt bei 29 $^o$C über 48 Stunden auf einem Rundschwingschütteltisch.

Mit je 800 ml der so erhaltenen Kulturlösung der ersten Vorzuchtstufe werden als zweite Vorzuchtstufe mit 150 l dampfsterilisiertem Medium (30 Minuten bei 120 $^o$C) der gleichen Zusammensetzung wie in der ersten Vorzuchtstufe gefüllte Edelstahlfermentoren (Bruttovolumen 240 l) beimpft. Die Kultivierung erfolgt über 40 Stunden bei 28 bis 29 $^o$C, einer Rührerdrehzahl von 240 U/min und einer Belüftungsrate von 1,0 VVM bei einem Überdruck von 0,03 MPa.

Zur Hauptkultur findet ein Edelstahlfermenter (Brutto-volumen 720 l) Verwendung, der mit 500 l Nährlösung folgender Zusammensetzung gefüllt ist (pro Liter): 60 g Maisstärke; 25 g Erdnußextraktionsschrot; 10 g Maisquellwasser (100 % Trockensubstanz) 10 g $(NH_4)_2SO_4$; 5 g Rügenkreide; 3 g Sonnenblumenöl; Leitungswasser ad 1000. Der pH-Wert der Nährlösung wird vor der Sterilisation auf pH 6,8 bis 7,0 eingestellt. Die Sterilisation erfolgt über 30 Minuten bei 120 $^o$C.

Der Fermentationsansatz wird mit 5 % Impfmaterial aus der zweiten Vorzuchtstufe beimpft. Im Verlaufe der Fermentation werden zusätzlich zweimal 30 g Maisstärke pro 1 Kultur als 30%ige Suspension zugesetzt (nach Verflüssigung der wäßrigen Aufschwemmung in an sich bekannter Weise durch Behandlung mit Enzympräparaten mit amylolytischer Aktivität, beispielsweise Brauerreienzym und anschließender Sterilisation). Die Azidität der Kulturlösung wird mittels 25%iger Ammoniumhydroxidlösung auf einen pH-Wert von 6,2 ± 0,1 konstant gehalten.

Nach Ablauf einer 144stündigen Fermentation bei 29 $^o$C, einer Rührergeschwindigkeit von 320 U/min und einer Belüftungsrate von 0,75 VVM bei einem Überdruck von 0,03 MPa werden 580 l Kulturlösung erhalten, in der eine Produktkonzentration von 6,85 ug Nourseothricin pro ml bestimmt wurde.

Beispiel 7

Für die Hauptkultur dient ein mit Vorrichtung zur Sterilbelüftung und einem zentral gelagerten Rührwerk versehener Edelstahlfermenter (4200 Liter Bruttovolumen), der mit 2500 Liter Nährmedium folgender, auf 1 Liter Leitungswasser bezogener Zusammensetzung gefüllt war: 32 g Kartoffelstärke, 60 g Glucose, 11 g Sojaextraktionsschrot, 11 g $(NH_4)_2SO_4$, 2 g $MgSO_4 \cdot 7H_2O$, 1 g NaCl, 6 g $CaCO_3$ und 0,5 g $ZnSO_4$. Als Entschäumer werden je Liter Kulturlösung 0,5 g Antaphron NM 40 zugesetzt. Die Azidität wird vor der Sterilisation auf den pH-Wert 6,2 eingestellt. Die thermische in situ-Sterilisation des Nährmediums (ohne Glucoseanteil) mit Direktdampf erfolgt auf der Basis des Sterilisationsfunktionalwertes als Regelgröße. Der Sollwert für diese Regelung liegt bei, $St = 35$ (Kontaminationsrisiko $10^{-3}$, Zelldichte $5 \cdot 10^{6}$ Zellen/ml KL vor der Sterilisation) und wird bei einer Plateautemperatur um 120 $^{o}$C durch Steuerung der Menge des zugeführten Direktdampfe eingehalten. Dabei wurde der aktuelle Wert des Sterilisationsfunktionals St gemäß

$$St = -\int_{0}^{t} f \, [A, E, T_{(\tau)}] \, d\tau$$

mit $f[-]$ als Arrhenius-Funktion aus dem aktuellen Verlauf der Sterilisationstemperatur T (t) ermittelt.

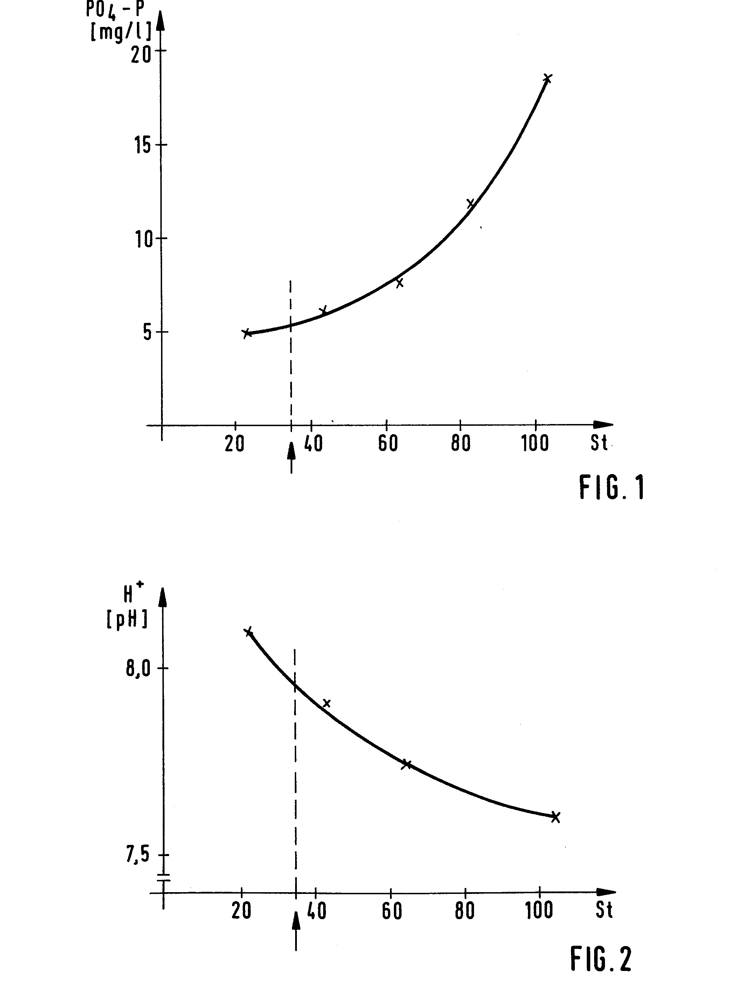

Die Abbildungen 1 und 2 zeigen die Abhängigkeit der Konzentration des säurelöslichen Phosphats im rückgekühlten sterilisierten Nährmedium und dessen Azidität vom Sollwert des Sterilisationsfunktionals, St = 35 entspricht der Einstellung der Startkonzentration des verfügbaren Phosphats auf 5 mg/l pH 8,0. Die Absenkung auf den physiologisch erforderlichen pH-Wert von 7,4 zu Fermentationsbeginn wird mittels Zugabe von Schwefelsäure realisiert, nachdem der in Form einer 50%igen wäßrigen Lösung getrennt sterilisierte Glucoseanteil (30 Minuten auf 120 $^{o}$C erhitzt) aseptisch zugesetzt war. Der mit 4 % Inoculum aus der zweiten Vorzuchtstufe beimpfte Fermentationsansatz der Hauptkultur wird 120 Stunden bei 29 $^{o}$C, einer Belüftungsrate von 1250 Liter pro Minute (0. bis 12. Stunde) bzw. 2500 Liter pro Minute (12. bis 120. Stunde) bei 0,03 Pa Überdruck unter Rühren mit 180 min$^{-1}$ kultiviert.

Den Fermentationsverlauf zeigen

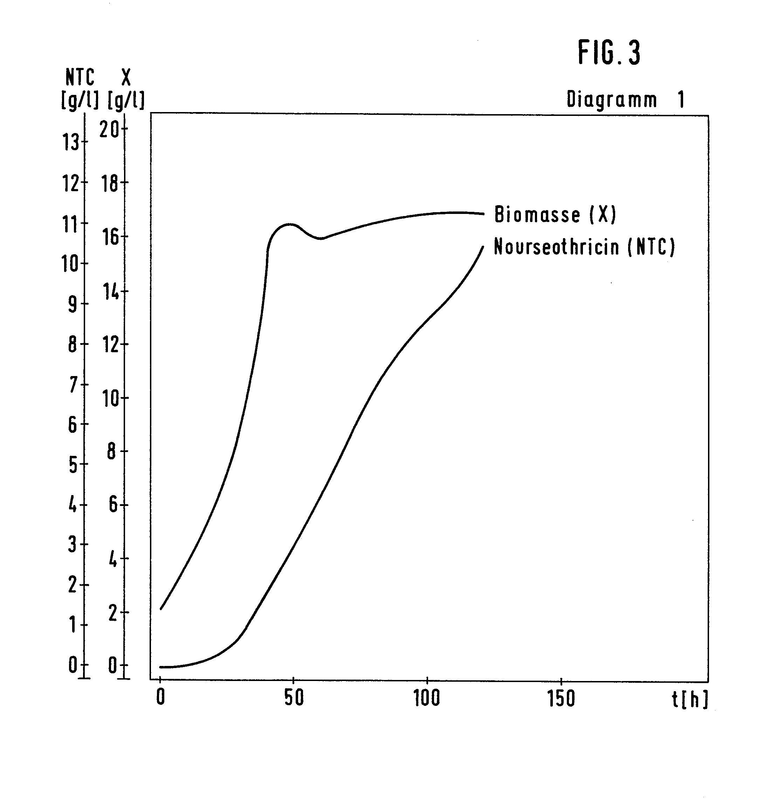

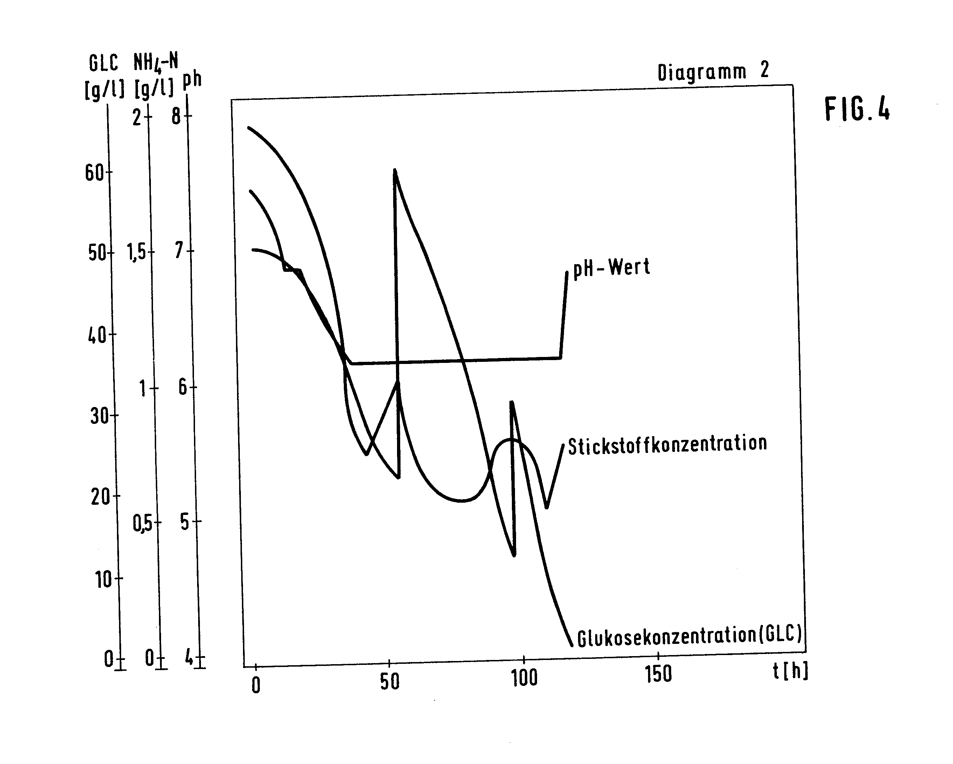

Abbildung 3  Zeitliche Verläufe des produzierten Antibiotikums Nourseothricin und des Trockengewichtes der Biomasse Abbildung 4  Zeitverläufe des pH-Wertes, der Glucose-
             und Ammoniumstickstoffkonzentration

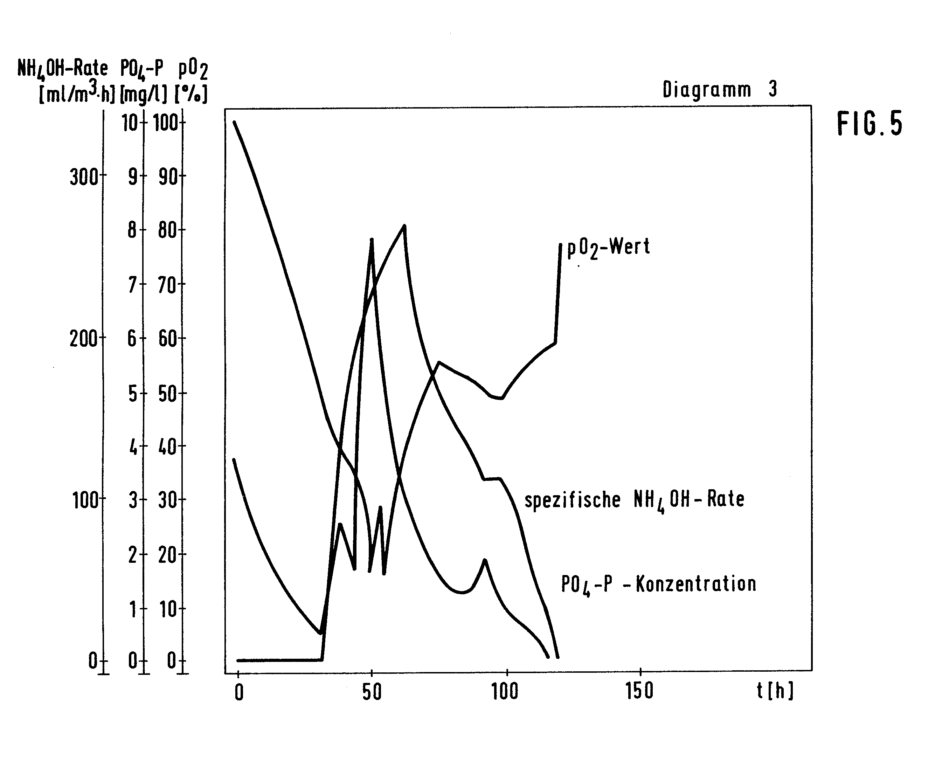

Abbildung 5  Zeitverhalten der Ammoniumhydroxid-Dosierrate, der Phosphatkonzentration und der
             Gelöstsauerstoffkonzentration

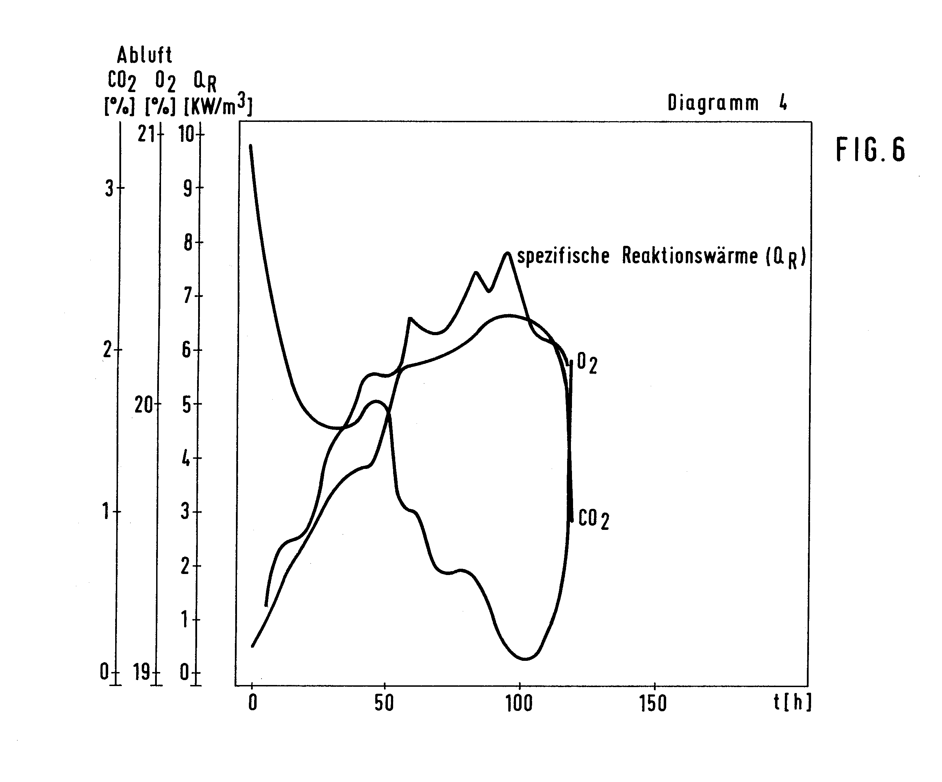

Abbildung 6  Zeitverläufe der Abgaszusammensetzung aus
             Sauerstoff und Kohlendioxid sowie der Reaktionswärme

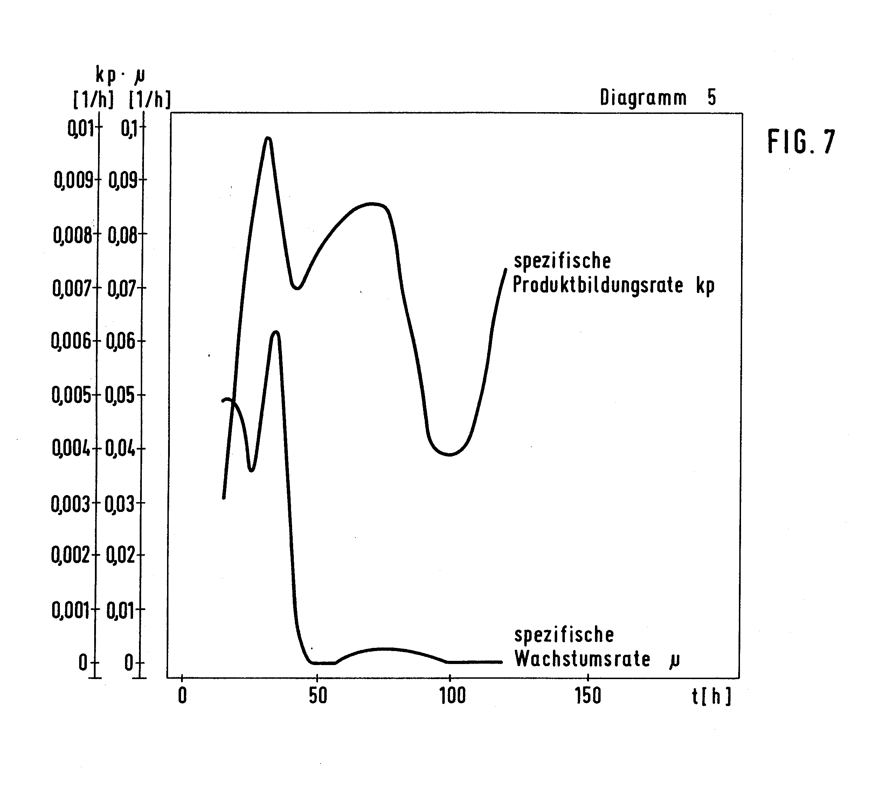

Abbildung 7  Zeitverläufe der spezifischen Produktbildungsrate und der spezifischen Wachstumsrate.

Die Fermentation wird manuell diskontinuierlich hinsichtlich der Regeleingriffe mittels direkter Phosphatdosierung und Glucosedosierung nach Maßgabe des Verhaltens
der Ammoniumhydroxid-Dosierrate geführt. Das pH-Sollwertintervall ist mit 6,2 $\pm$ 0,05 festgelegt und die untere
Grenze pH 6,15 wird nach 38 Stunden Fermentationszeit erreicht.

Zur direkten Phosphat-Dosierung wird eine sterile wäßrige
Lösung von $KH_2PO_4$ benutzt. Während der ersten 32 Stunden
wird diese Lösung so zudosiert, daß zur Anregung der Stoffwechselaktivität insgesamt pro Liter Kulturlösung 42 mg
Phosphor in Form von Phosphat eingesetzt werden.
Festgelegt durch das gewählte pH-Sollwertintervall, den
Pufferungszustand des Nährmediums und die Lauge-Konzentration setzt ab der 38. Stunde die Ammoniumhydroxid
(25%ig)-Dosierung ein.
Die beobachtete Verringerung des Anstiegs der Ammoniumhydroxid-Dosierrate löst direkte Phosphatdosierungen von
jeweils 8,5 mg Phosphat-Phosphor pro Liter Kulturlösung
zwischen der 47. und 48. Stunde bzw. 53. und 54. Stunde
aus.
Dadurch erreicht die Dosierrate ihren Maximalwert zur 62.
Stunde und fällt dann dem Übergang von direkter zur indirekter Phosphatdosierung, durch Freisetzen aus Substraten, entsprechend ab. Auf diese Weise befindet sich die
Fermentation etwa 20 Stunden in der Nähe der optimalen

spezifischen Dosierrate von 230 ml 25%iger Ammoniumhydroxidlösung pro Kubikmeter Kulturlösung und Stunde. Die zugegebene Ammoniumhydroxid-Lösung ist ausreichend, um die Stickstoffkonzentration im physiologisch günstigen Bereich zu halten. Deshalb werden keine weiteren Stickstoffquellen zudosiert. Bezüglich der Kohlenstoffquelle ist die nach der zweiten Phosphatdosierung verstärkte Verringerung des Anstiegs der Ammoniumhydroxid-Dosierrate Anlaß zur Zudosierung von 48 g Glucose pro Liter Kulturlösung zur 56. Stunde. Die Indikation des Übergangs zur indirekten Phosphatdosierung um die 95. Stunde wird zur Auslösung der zweiten Glucosedosierung von 18 g Glucose pro Liter Kulturlösung verwendet.

Bei der direkten Phosphatdosierung ergibt sich hinsichtlich der Konzentration des Gelöstsauerstoffe keine Beschränkung.
Reaktionswärme und Abluftzusammensetzung weisen den Stand der Fermentationsphasen aus und dienen zur Vermeidung von Substratlimitationen. Der Nourseothricin-Endwert von 10,5 $\mu$g/ml Kulturlösung wird erreicht, indem die effektive spezifische Produktbildungsrate über den Zeitraum von 45 Stunden zwischen $8 \cdot 10^{-3}$ pro Stunde und $9 \cdot 10^{-3}$ pro Stunde liegt. Außerdem wird die eingesetzte Glucose voll ausfermentiert und das aufarbeitungstechnisch gesetzte Biomasselimit nicht überschritten.


Beispiel 8

Zur Isolierung des Antibiotikums werden 750 l Fermentationslösung mit einem Gehalt von 2900 $\mu$g/ml Nourseothricinbase mit einer gesättigten wäßrigen Lösung von 7,6 kg Oxalsäure versetzt. Nach Einstellung von pH 4,2 mit Ammoniak wird die Lösung kurzzeitig auf 70 $^{o}$C erhitzt, der Feststoffanteil durch Separation abgetrennt, das Kulturfiltrat mit Ammoniak neutralisiert, zur Entfernung von Trübstoffen erneut separiert und zur Adsorption mit einer Geschwindigkeit von 25 l/Stunde durch zwei hinter-

einander geschaltete Säulen mit je 5 l Wofatit OP (Na-Form) von unten nach oben geleitet. Die Adsorbate der beiden Säulen werden nacheinander mit je 30 l Wasser, je 20 l 0,1 N Essigsäure und je 15 l Wasser gewaschen. Danach wird das Antibiotikum mit je 20 l 0,5 N Schwefelsäure und anschließend mit je 10 l Wasser eluiert.

Eluate mit pH-Werten unter 3,0 werden mit Wofatit AD 41 (OH-Form) neutralisiert.

Das Eluat der Säule I (32,8 l) wird unter Rühren solange mit Diammoniumhydrogenphosphat und Ammoniak versetzt, bis bei pH-Werten von 7,5 bis 7,7 kein Niederschlag mehr entsteht. Nach Abtrennung der ausgefallenen anorganischen Phosphate durch Filtration wird das klare Filtrat zur Entsalzung mit einer Geschwindigkeit von 20 l/Stunde über 10 l Wofatit KP 16 (H-Form) und anschließend über 10 l Wofatit AD 41 (OH-Form) filtriert. Nach dem Waschen der Austauscherharze mit 20 l Wasser werden Eluat und Waschwasser vereinigt (ca. 55 l), im Vakuum bei 30 bis 40 °C auf 6,5 l konzentriert und mit 100 g Aktivkohle behandelt. Das Filtrat wird mit Schwefelsäure auf pH-Werte von 4,0 bis 4,5 eingestellt und anschließend unter Rühren in 75 l Methanol eingetropft, wobei amorphes Nourseothricinsulfat ausfällt. Der Niederschlag wird abfiltriert, mit Methanol gewaschen und im Vakuum bei 40 °C getrocknet.

Mit dem Eluat von Säule II wurde in gleicher Weise verfahren.

Ausbeute:

Säule I: 745 g Nourseothricin-Sulfat:

$$[\alpha]_D^{25} = -27,5^{\circ} \ (c = 1; \text{Wasser}); \text{Sulfatasche};$$

2,2 % Mikrobiologische Wirksamkeit: 687 µg/mg;

Säule II: 579 g Nourseothricin-Sulfat; Sulfatasche: 2,0 % Mikrobiologische Wirksamkeit: 470 µg/mg;

Beispiel 9

9,6 l einer nach Beispiel 8 erhaltenen und entsalzten Eluatfraktion werden im Vakuum bei 30 bis 40 $^\circ$C auf ein Volumen von 440 ml eingedampft, das Eluatkonzentrat mit 12 g Aktivkohle behandelt und nach Filtration mit Schwefelsäure auf pH 6,0 eingestellt.

Sprühtrocknung:

220 ml des entfärbten Eluatkonzentrates (Feststoffgehalt 33 %) werden in einem Laborsprühtrockner Mini Spray Dryer Büchi 190 bei einer Lufteintrittstemperatur von 165 bis 175 $^\circ$C und einer Austrittstemperatur von 85 bis 90 $^\circ$C sprühgetrocknet.

Ausbeute:  58,4 g Nourseothricin-Sulfat
Mikrobiologische Wirksamkeit 980 µg/mg
$[\alpha]_D^{25}$ = -32,8$^\circ$ (c = 1; Wasser); Sulfatasche
0,4 %; Wassergehalt: 6,0 %
C 36,87, 37,01; H 6,48, 6,66; N 16,99, 16,95; S 5,64, 5,71;

Gefriertrocknung:

220 ml des entfärbten Eluatkonzentrates werden bei einer Produkt-Ausgangstemperatur von -20 $^\circ$C und einem Enddruck von 0,03 Torr in einer Gefriertrocknungsanlage TG 5 (Hersteller: VEB Hochvakuum Dresden) getrocknet.

Ausbeute:

71,5 g Nourseothricin-Sulfat
Mikrobiologische Wirksamkeit: 994 µg/mg
$[\alpha]_D^{25}$ = -34,2$^\circ$ (c = 1,28; Wasser) Wassergehalt: 3,0 %;
C 36,85, 36,98; H 6,32, 6,32; N 16,89, 17,16; S 5,87, 5,85;

Die Elementaranalyse entspricht annähernd der folgenden Zusammensetzung eines 2 : 1 Gemisches von Streptothricin

0136256

D- und Streptothricin F-Sulfat: $(2C_{31}H_{58}N_{12}O_{10} \cdot 2H_2SO_4 \cdot$

$2H_2O + C_{19}H_{34}N_8O_8 \cdot H_2SO_4 \cdot 2H_2O)$

## Beispiel 10

Zur Adsorption des Antibiotikums werden 75 l eines nach Beispiel 8 erhaltenen Kulturfiltrates mit 4846/ug/ml Nourseothricinbase aufsteigend mit einer Geschwindigkeit von 4 l/Stunde durch eine Säule mit 2 l Wofatit CP (Na-Form) geleitet. Das Harz wird anschließend der Reihe nach mit 15 l aqua dest., 9 l 0,1 N Essigsäure und 10 l aqua dest. gewaschen und danach das Antibiotikum mit 14 l 0,5 n Phosphorsäure eluiert. Das Eluat (pH 3 bis 4) wird durch Ausrühren mit 2 l Wofatit AD 41 (OH-Form) neutralisiert. Ausgefallenes Kalziumphosphat mit zusammen mit dem Ionenaustauscher abfiltriert und das Filtrat mit Ammoniak bis pH 7,7 versetzt, wobei Ammoniummagnesiumphosphat ausfällt und durch Filtration entfernt wird. Anschließend werden einwertige Kationen durch Ausrühren mit 1 l Wofatit KP 16 (H-Form) aus der Nourseothricinphosphatlösung abgetrennt. Danach wird die Lösung (17 l) im Vakuum bei 40 $^{\circ}$C auf 1,5 l eingedampft, mit Phosphorsäure bis pH 4,5 angesäuert und mit 7,5 g Aktivkohle behandelt. Das klare Filtrat wird in 20 l Methanol eingetropft. Kolloidal in Lösung bleibendes Nourseothricinphosphat wird durch Zugabe von 25%iger Dimethylaminlösung zur Ausflockung und in eine gute filtrierbare Form gebracht. Der Niederschlag wird abfiltriert, mit Methanol gewaschen und im Vakuum über Kalziumchlorid getrocknet.

Ausbeute: 169,5 g Nourseothricin-Phosphat"

$[\alpha]_D^{25}$ = -29,9$^\circ$ (c = 1; Wasser); Sulfatasche;

106 %

Mikrobiologische Wirksamkeit: 818 µg/mg

Beispiel 11

75 l einen nach Beispiel 8 erhaltenen Kulturfiltrates mit 3000 ug/ml Nourseothricinbase werden mit einer Geschwindigkeit von 4 l/Stunde aufsteigend durch eine Säule mit 2 l Wofatit OP (Na-Form) geleitet. Nach dem Waschen des Harzes mit 15 l aqua dest., 10 l 0,1 N Essigsäure und nochmals mit 10 l aqua dest. wird das Antibiotikum mit 10 l 0,5 N Oxalsäure eluiert. Das Eluat (11 l) wird durch Ausrühren mit 2 l Wofatit AD 41 (OH-Form) neutralisiert, ausgefallene Salze und Ionenaustauscher gemeinsam abfiltriert und das klare Filtrat zur Entfernung einwertiger Kationen mit 2 l Wofatit KP 16 (H-Form) ausgerührt. Anschließend wird die Lösung mit 0,5 l Wofatit AD 41 (OH-Form) neutralisiert und im Vakuum bei 40 °C auf 800 ml eingedampft. Das Konzentrat wird mit 7 g Aktivkohle behandelt und nach Filtration in 12 l Methanol eingetropft. Kolloidal in Lösung bleibendes Nourseothricin-Oxalat wird durch Zugabe von 25%iger Dimethylaminlösung ausgeflockt und in eine gut filtrierbare Form gebracht. Der abfiltrierte Niederschlag wird mit Methanol gewaschen und im Vakuum über Calziumchlorid getrocknet.

Ausbeute:

162 g Nourseothricin-Oxalat

$[\alpha]_D^{25}$ = -41,8° (c = 1; Wasser); Sulfatasche: 0,9 %

Mikrobiologische Wirksamkeit: 809 µg/mg

Beispiel 12

In Schweinemastversuchen konnte durch Verabreichung eines mit 40 mg/kg Trockensubstanz (TS) Nourseothricin-Hydrochlorid supplementiertes Mischfuttermittel (Prämix) während der 70 Versuchstage eine um 6 % verbesserte durchschnittliche Lebendmasse-Entwicklung ermittelt werden. Durch Verabreichung von 21 mg/kg TS Nourseothricin als Myzeladsorbat mit der Futterration konnte während des 70tägigen Mastversuches die durchschnittliche tägliche Lebendmasse-Zunahme um 23 % erhöht werden, wobei der Futteraufwand bis zu 7 % geringer war als bei den Tieren der Kontrollgruppe.

Die Verabreichung von Nourseothricin in Form von Myzel-adsorbat in gestaffelten Dosierungen bis zu 52,5 mg Wirkstoff/Tier/Tag an Kälber übte einen sehr günstigen Einfluß auf die Tiergesundheit aus, erkennbar an einen deutlichen Rückgang der Verendungen und einer drastischen Senkung der Durchfall-Tage. Die günstigen Wirkungen auf die Lebendmasse-Zunahme von Kälbern waren insbesondere während der ersten vier Lebenswochen festzustellen. Tägliche Zulagen von 35 mg/Tier Nourseothricin zum Futter bewirkten nach 4wöchiger Mastdauer eine Verbesserung der Lebendmasse-Entwicklung um 25 %; nach Abschluß des 10-wöchigen Mastversuches betrug dieser Wert maximal 13 %.

Verträglichkeit von Nourseothricin-Myzel erwies sich im Tierversuch als untoxisch. Dosen bis 5000 mg/kg Körpermasse (KM), einmalig mittels Schlundsonde an männliche und weibliche Wistarratten (Körpermassen ca. 200 g) verabfolgt, beeinflußten weder die Körpermassenentwicklung der Versuchstiere noch wurde substanzbedingte Sterblichkeit beobachtet.


Beispiel 12a

Zur Herstellung eines Nourseothricin-haltigen Prämix in Form von myzelhaltigem Bentonit-Adsorbat werden unmittelbar nach Abbruch der Fermentation 0,1 Liter Formalin-Lösung pro 100 Liter Kulturlösung zugegeben und 15 bis 30 Minuten verrührt. Anschließend wird 25%ige Schwefelsäure zudosiert, bis die Azidität von pH 6,0 erreicht ist.

Mit entsprechendem zeitlichen Vorlauf wird eine Bentonit-Suspension in einem Rührkessel aus säurebeständigem Material vorbereitet durch Eintragen von 2 kg natrifiziertem Bentonit in 15 Liter Wasser und Zusatz von 0,2 Liter einer 25%igen Schwefelsäure. Die Quelldauer dieser Suspension muß mindestens 12 Stunden betragen.

Nach dem Einrühren der so vorbereiteten Bentonit-Suspension in die 100 Liter Kulturlösung (Wirkstoffgehalt: 5000 mg Nourseothricin je Liter) stellt sich in der Regel ein pH-Wert zwischen 6,0 bis 6,5 ein; erforderlichenfalls ist durch Zusatz von 25%iger Schwefelsäure bzw. 30%iger Natronlauge auf diesen pH-Bereich einzustellen. Während des Adsorptionsprozesses ist die Reaktionslösung ständig zu rühren. Nach mindestens 60minütiger Reaktionszeit kann mit der Abtrennung des Feststoffes begonnen werden, was durch Filtration oder Separation geschehen kann.

Für derartige Trennarbeiten eignet sich besonders gut ein Vakuum-Drehfilter, das mit entsprechenden zeitlichen Vorlauf durch Aufziehen einer Filterhilfsmittelschicht aus einen speziell zubereitetem Calciumsulfat vorzubereiten ist.

Bei einem Unterdruck von $7 \cdot 10^4$ Pa können Filtrierleistungen von 250 $1/m^2 \cdot h$ erzielt werden. Aus 100 Liter Kulturlösung ($\hat{=}$ etwa 115 Liter Bentonit-Kulturlösung-Suspension) werden 93 bis 98 Liter Filtrat mit einem Rest-Wirkstoffgehalt entsprechend 5 bis 10 % der Ausgangsaktivität.

Der als Zwischenprodukt abfiltrierte Feststoff beträgt 12 bis 14 kg bei einer Restfeuchte von 58 bis 65 %. Bei der Filtration von 110 bis 115 Liter Suspension werden 2,0 bis 2,5 kg Filterhilfsmittel verbraucht. Der abgetrennte, feuchte Feststoff ist umgehend der Trocknung zuzuführen. Während des Trockenprozesses im Warmluftstrom darf die Produkttemperatur 70 °C nicht überschreiten. Aus 12 bis 14 kg feuchtem Feststoff werden 4,5 bis 5,0 Trockenprodukt mit einem Nourseothricin-Gehalt von 75 bis 85 g je kg Trockensubstanz erhalten. Der Wirkstoffgehalt wurde mittels des Agarplatten-Diffusionstest unter Verwendung

von Bacillus subtilis ATCC 6633 als Testkeim bestimmt.

Bei Verwendung eines Ringstromtrockners fällt das Trockenprodukt so feinkörnig an, daß es unmittelbar als Prämix zur Dosierung in das Mischfuttermittel eingesetzt werden kann. Das mittels Horden-, Band- oder Wirbelschichttrockner gewonnene Produkt muß vor der Standardisierung bzw. Einmischung vermahlen werden.

Die Dosierung der Mischfuttermittel wird so bemessen, daß der Nourseothricin-Gehalt 5 bis 100 mg je kg Mischfuttermittel-Trockensubstanz beträgt.

Die Zusammensetzung der für die einzelnen landwirtschaftlichen Nutztierarten und Mastperioden bestimmten Mischfuttermittel wird durch "Qualitätsanforderungen für Mischfuttermittel in der Tierproduktion" verbindlich geregelt.

Beispiel 13

Zur Herstellung eines Nourseothricin-haltigen Prämix in Form von myzelhaltigem Trockenprodukt wird unmittelbar nach Beendigung der Fermentation die Kulturlösung, wie im Beispiel 12 beschrieben, mit Formalin versetzt und durch Zudosierung von 25%iger Schwefelsäure auf die Azidität von pH 6,0 eingestellt. Anschließend wird die so vorbehandelte Kulturlösung unter schonenden Bedingungen in einem geeigneten Verdampfer (Vakuumumlaufverdampfer, Unterdruck $4 \cdot 10^4$ bis $6 \cdot 10^4$ Pa, Temperatur 70 °C) eingeengt und das Konzentrat anschließend mittels eines Vakuumwalzentrockners bis zum feinscholligen Festprodukt getrocknet. Während des bei einem Unterdruck von $4 \cdot 10^4$ bis $6 \cdot 10^4$ Pa ablaufenden Trockenprozesses darf das zu trocknende Gut nicht über 70 °C erhitzt werden; die Restfeuchte des getrockneten Endproduktes muß geringer sein als 3 %. Aus 100 Liter Kulturlösung mit einem ursprünglichen Wirkstoffgehalt von 5000 mg/l Nourseothricin werden 3750 g myzelhaltiges Trockenprodukt mit einem Wirkstoffgehalt von 113 mg Nourseothricin je g Trockensubstanz erhalten.

Das so erhaltene myzelhaltige Trockenprodukt ist vor der Standardisierung bzw. Einmischung zu vermahlen.

Die Verfahren zur mikrobiologischen Bestimmung des Wirkstoffgehalts und der Dotierung von Mischfuttermitteln sind die gleichen wie im Beispiel 12 beschrieben.

Die Vorteile der Erfindung werden nachstehend bezüglich ihrer Anwendung dargestellt.

Für einen in Bodenhaltung durchgeführten Fütterungsversuch wurden 400 unsortierte Broiler-Küken (Tetra B) in Untergruppen mit je 8 Tieren aufgeteilt. Je Behandlung erfolgten 10 Wiederholungen. Die mittlere Küken-Masse bei Versuchsbeginn betrug 38 g/Tier. Verabreicht wurde ein den staatlichen Qualitätsförderungen 1980/81 entsprechendes Broilermasteinstellfutter bestehen aus:

| | |
|---|---|
| 64,5 % Maisschrot | 2,5 % Mineralstoffmischung für Geflügel mit 54 g P/kg |
| 21,0 % Sojaextraktionsschrot | |
| 6,0 % Fischmehl | |
| 2,0 % Futterhefe | 1,0 % Wirkstoffvormischung für Broilermasteinstellfutter (antibiotikafrei) |
| 3,0 % Raps | |

Die Futterrationen für die Versuchsgruppen wurden mit Reinsubstanz von Nourseothricin-Hydrochlorid supplementiert.

Die Wirkung gestaffelter Nourseothricin-Zusätze auf die Lebendmasse-Entwicklung, den Futterverzehr und den Futteraufwand wird in den Tabellen 5, 6 und 7 dargestellt.

Für einen in Batteriehaltung durchgeführten Fütterungsversuch wurden 3000 unsortierte Broiler-Küken (Tetra B) in Untergruppen mit je 100 Tieren aufgestellt. Je Behandlung erfolgten 6 Wiederholungen. Die mittlere Kükenmasse bei Versuchsbeginn betrug 37,5 g. Das verabreichte Futter hatte die gleiche Zusammensetzung wie oben angegeben. Die Futterrationen für die Versuchsgruppen wurden mit Reinsubstanz von Nourseothricin-Hydrochlorid supplementiert.

Die Wirkung gestaffelter Nourseothricin-Zusätze auf die Lebendmasse-Entwicklung, den Futterverzehr und den Futteraufwand wird in den Tabellen 8, 9 und 10 dargestellt.

Für einen in Bodenhaltung durchgeführten Fütterungsversuch wurden 55 Ferkel von gegenwärtig in der Schweineproduktion verwendeten Kreuzungstieren in Untergruppen mit je 11 Tieren aufgeteilt. Die mittlere Lebendmasse bei Versuchsbeginn betrug 10,5 kg/Tier.

Die eingesetzten Mischfuttermittel hatten folgende Zusammensetzung:

Bis 40 kg Lebendmasse:
45,0 % Weizen
33,0 % Gerste
5,0 % Fischmehl
.5,0 % Trockenmagermilch
10,0 % Sojaextraktionsschrot
1,0 % Mineralstoffvormischung mit 50 g P/kg
1,0 % Wirkstoffvormischung für Ferkelaufzucht-
     futter I (antibiotikafrei)

Ab 40 kg Lebendmasse:
40,0 % Weizen
48,0 % Gerste
7,0 % Sojaextraktionsschrot
3,0 % Fischmehl
1,0 % Mineralstoffvormischung mit 35 g P/kg
1,0 % Wirkstoffvormischung für Schweinemast-
     futter II (antibiotikafrei)

Die Futterrationen für die Versuchsgruppen wurden mit Reinsubstanzen von Nourseothricin-Hydrochlorid, Kormogrisin bzw. Zinkbacitracin supplementiert.

Die Wirkung gestaffelter Ergotropika-Zusätze auf die Lebendmasse-Entwicklung, die Futteraufnahme und den Futteraufwand wird in den Tabellen 11, 12 und 13 dargestellt.

Für zwei in Bodenhaltung durchgeführten Fütterungsversuche (Versuche A und B) wurden jeweils 48 Ferkel von gegenwärtig in der Schweineproduktion verwendeten Kreuzungstieren in Untergruppen mit je 12 Tieren aufgeteilt. Die mittlere Lebendmasse bei Versuchsbeginn betrug 10,7 kg (Versuch A) bzw. 12,6 kg (Versuch B).

Die Futterrationen für die Versuchsgruppe wurden mit einem Nourseothricin-Prämix in Form von myzelhaltigem Bentonit-Adsorbat supplementiert.

Die im Verlaufe der jeweils 70tägigen Versuchsdauer ermittelten Wirkungen auf die mittlere Lebendmasse-Entwicklung, den Futterverzehr und den Futteraufwand werden in den Tabellen 14, 15 und 16 dargestellt.

Für einen 10wöchigen Fütterungsversuch wurden 36 Käber (schwarzbuntes Milchrind) in Untergruppen mit je 9 Tieren aufgeteilt. Die mittlere Lebendmasse bei Versuchsbeginn betrug 54 kg/Tier.

Die Milchaustauschertränke enthielt je Liter 100 g das in Wasser gelösten Milchaustauschers für Kälber. Diese Tränke wurde unmittelbar vor der Verabreichung mit myzelhaltigem Nourseothricin-Bentonit-Adsorbat supplementiert.

Zusätzlich erhielten die Tiere ad libitum eine Konzentratmischung folgender Zusammensetzung:

    30,0 % Weizenschrot
    38,0 % Gerstenschrot
    20,5 % Sojaextraktionsschrot
     4,0 % Zuckerrübenschnitzel, getrocknet
     3,0 % Futterhefe, getrocknet
     1,5 % Mileipan (Vitamin-Konzentrat)

sowie dem Alter entsprechend täglich bis zu 0,5 kg Heu/Tier.

Die Wirkung gestaffelter Nourseothricin-Zusätze auf die Tiergesundheit und die Lebendmasse-Entwicklung bei Kälbern wird in der Tabelle 13 dargestellt.

Tabelle 1: Förderung der Nourseothricin-Bildung in Schüttelkulturen des Stammes Streptomyces noursei ZIMET 43716 durch Zusätze von Natriumazid, Brenzkatechin, Amytal, Zinksulfat, ß-Alanin, Medium Bo-30

| Konzentration des Zusatzes (mM) | $\mu$g/ml Nourseothricin | | | | |
|---|---|---|---|---|---|
| | Natriumazid | Brenzkatechin | Amytal | Zinksulfat | ß-Alanin |
| 0,025 | 183 | – | – | – | – |
| 0,050 | 230 | – | – | – | – |
| 0,075 | 291 | – | – | – | – |
| 0,100 | 330 | – | – | – | – |
| 0,125 | 458 | – | – | – | – |
| 0,150 | 544 | – | – | 388 | – |
| 0,175 | 372 | – | – | 399 | – |
| 0,200 | 230 | – | – | 440 | – |
| 0,250 | 224 | 263 | 185 | 469 | 210 |
| 0,500 | – | 277 | 221 | 560 | 329 |
| 0,750 | – | 294 | 228 | 702 | 311 |
| 1,00 | – | 396 | 291 | 552 | 258 |
| 1,25 | – | 412 | 301 | 439 | 255 |
| 1,50 | – | 382 | 206 | 437 | 212 |
| 1,75 | – | 250 | 174 | 124 | 157 |
| Ohne Zusatz (Kontrolle) | | | 95 | | |

Tabelle 2: Förderung der Nourseothricin-Bildung in Schüttelkulturen des Stammes Streptomyces noursei ZIMET 43716 durch Zusätze von Natriumazid, Brenzkatechin bzw. Zinksulfat, Medium Bo-31

| Konzentration des Zusatzes (mM) | ug/ml Nourseothricin | | |
|---|---|---|---|
| | Natriumazid | Brenzkatechin | Zinksulfat ($ZnSO_4 \cdot 7H_2O$) |
| 0,050 | 961 | – | – |
| 0,100 | 1135 | – | – |
| 0,125 | 1426 | – | – |
| 0,150 | 1891 | – | – |
| 0,175 | 1085 | – | – |
| 0,200 | 657 | 941 | – |
| 0,300 | – | 1401 | 887 |
| 0,400 | – | 1984 | 1711 |
| 0,500 | – | 1166 | 1934 |
| 0,750 | – | 986 | 2158 |
| 1,00 | – | 868 | 2585 |
| 1,25 | – | 713 | 2604 |
| 1,50 | – | – | 2108 |
| 2,00 | – | – | 781 |
| Ohne Zusatz (Kontrolle) | | 620 | |

Tabelle 3:

Nourseothricin-Gehalte in mit $KH_2PO_4$, Glucose und $(NH_4)_2SO_4$
dosierten und in nichtdosierten Laborfermenterkulturen
des Stammes Streptomyces noursei ZIMET 43716 / NG13-14 in
Abhängigkeit von der Fermentationszeit

| Fermentations-zeit h | Mit Dosierung /ug/ml | % | Ohne Dosierung (Kontrolle) /ug/ml | % |
|---|---|---|---|---|
| 24 | 1035 | 249 | 415 | 100 |
| 48 | 2980 | 165 | 1805 | 100 |
| 72 | 5412 | 294 | 1840 | 100 |
| 96 | 8311 | 213 | 3905 | 100 |
| 120 | 8800 | 195 | 4520 | 100 |
| 144 | 9230 | 213 | 4005 | 100 |

0136256

Tabelle 4: Nourseothricin-Gehalte in mit $KH_2PO_4$,
Glucose und $(NH_4)_2SO_4$ dosierten und nichtdosierten 500 l-Fermenterkulturen des Stammes
Streptomyces noursei ZIMET 43716 / NG13-14
in  Abhängigkeit von der Fermentationszeit

| Fermentations-zeit | Mit Dosierung | | Ohne Dosierung (Kontrolle) | |
|---|---|---|---|---|
| h | µg/ml | % | µg/ml | % |
| 20 | 225 | 216 | 104 | 100 |
| 44 | 1600 | 228 | 702 | 100 |
| 68 | 4858 | 299 | 1627 | 100 |
| 92 | 4903 | 208 | 2362 | 100 |
| 116 | 6862 | 191 | 3585 | 100 |
| 144 | 7781 | 200 | 3885 | 100 |

0136256

Tabelle 5: Einfluß gestaffelter Nourseothricin-Zusätze auf
Lebendmasse-Entwicklung bei Broiler-Küken in
Bodenhaltung

| Nourseothricin-Hydrochlorid-Supplement (mg/kg) | Lebendmasse je Tier | | | | | |
|---|---|---|---|---|---|---|
| | 14. Tag | | 28. Tag | | 52. Tag | |
| | (g) | (%) | (g) | (%) | (g) | (%) |
| 0 | 200 | 100 | 525 | 100 | 1463 | 100 |
| 5,0 | 190 | 95 | 532 | 101 | 1469 | 100 |
| 10,0 | 208 | 104 | 548 | 104 | 1478 | 101 |
| 30,0 | 210 | 105 | 593 | 113 | 1548 | 106 |
| 50,0 | 214 | 107 | 588 | 112 | 1543 | 106 |
| $T_{0,05}$ | 14 | | 51 | | 77 | |

Tabelle 6: Einfluß gestaffelter Nourseothricin-Zusätze auf
den Futterverzehr bei Broiler-Küken in Bodenhaltung

| Nourseothricin-Hydrochlorid-Supplement (mg/kg) | Futterverzehr/Tier | | | |
|---|---|---|---|---|
| | 28. Tag | | 52. Tag | |
| | (g) | (%) | (g) | (%) |
| 0 | 1105 | 100 | 3560 | 100 |
| 5,0 | 1087 | 98 | 3430 | 96 |
| 10,0 | 1183 | 107 | 3505 | 99 |
| 30,0 | 1146 | 104 | 3522 | 99 |
| 50,0 | 1100 | 100 | 3476 | 98 |

Tabelle 7: Einfluß gestaffelter Nourseothricin-Zusätze auf den Futteraufwand (1. bis 52. Masttag) bei Broiler-Küken in Bodenhaltung

| Nourseothricin-Hydrochlorid-Supplement | 0 | 5,0 | 10,0 | 30,0 | 50,0 |
|---|---|---|---|---|---|
| Futteraufwand (kg/kg Zunahme) | 2,5 | 2,4 | 2,4 | 2,3 | 2,3 |
| (%) | 100 | 96 | 96 | 92 | 92 |
| $T_{0,05}$ | | | 0,2 | | |

Tabelle 8: Einfluß gestaffelter Nourseothricin-Zusätze auf die Lebendmasse-Entwicklung bei Broiler-Küken in Batteriehaltung

| Nourseothricin-Hydrochlorid-Supplement (mg/kg) | Lebendmasse je Tier | | | | | |
|---|---|---|---|---|---|---|
| | 14. Tag | | 28. Tag | | 52. Tag | |
| | (g) | (%) | (g) | (%) | (g) | (%) |
| 0 | 203 | 100 | 575 | 100 | 1477 | 100 |
| 20 | 196 | 97 | 594 | 103 | 1489 | 101 |
| 30 | 215 | 106 | 603 | 105 | 1567 | 106 |
| 40 | 219 | 108 | 614 | 108 | 1612 | 109 |
| 50 | 208 | 103 | 547 | 95 | 1544 | 105 |
| $T_{0,05}$ | | | 25 | | 114 | |

0136256

Tabelle 9: Einfluß gestaffelter Nourseothricin-Zusätze auf den Futterverzehr bei Broiler-Küken in Batteriehaltung

| Nourseothricin-Hydrochlorid-Supplement (mg/kg) | Futterverzehr/Tier | | | |
|---|---|---|---|---|
| | 28. Tag | | 52. Tag | |
| | (g) | (%) | (g) | (%) |
| 0 | 1131 | 100 | 3562 | 100 |
| 20 | 1186 | 105 | 3605 | 102 |
| 30 | 1157 | 102 | 3641 | 102 |
| 40 | 1048 | 93 | 3530 | 99 |
| 50 | 1061 | 94 | 3400 | 96 |

Tabelle 10: Einfluß gestaffelter Nourseothricin-Zusätze auf den Futteraufwand (1. bis 52. Masttag) bei Broiler-Küken in Batteriehaltung

| Nourseothricin-Hydrochlorid-Supplement (mg/kg) | 0 | 20 | 30 | 40 | 50 |
|---|---|---|---|---|---|
| Futteraufwand (kg/kg Zunahme) | 2,5 | 2,5 | 2,4 | 2,2 | 2,3 |
| (%) | 100 | 100 | 96 | 88 | 92 |

Tabelle 11: Wirkung verschiedener Ergotropika auf die tägliche Lebendmasse-Entwicklung bei Schweinen in
Bodenhaltung

| Lebendmasse-Abschnitt (Ergotropikum) | 3 bis 45 kg | | 45 bis 120 kg | | 8 bis 120 kg | |
|---|---|---|---|---|---|---|
| | (g) | (%) | (g) | (%) | (g) | (%) |
| Zinkbacitracin 120 mg/kg | 510 (82,8) | 105 | ./. | | | |
| Zinkbacitracin 40 mg/kg | ./. | | 621 (86,0) | 101 | 581 (70,7) | 101 |
| Kormogrisin 40 mg/kg | 559 (61,3) | 115 | 578 (74,4) | 94 | 574 (49,8) | 101 |
| Nourseothricin-Hydrochlorid-10 mg/kg | 505 (57,8) | 104 | 602 (55,0) | 98 | 567 (40,2) | 100 |
| Nourseothricin-Hydrochlorid 40 mg/kg | 587 (36,6) | 121 | 617 (63,7) | 101 | 602 (47,7) | 106 |
| Kontrolle | 484 | 100 | 613 | 100 | 568 | 100 |

(Zahlenwerte in Klammern ≙ Streuung der Messwerte)

Tabelle 12: Wirkung verschiedener Ergotropika auf die tägliche Futteraufnahme bei Scheinen in Bodenhaltung

| Lebendmasse-Abschnitt | 8 bis 45 kg | | 45 bis 120 kg | | 8 bis 120 kg | |
|---|---|---|---|---|---|---|
| (Ergotropikum) | (g) | (%) | (g) | (%) | (g) | (%) |
| Zinkbacitracin 120 mg/kg | 1,33 | 95 | ./. | | 2,12 | 95 |
| Zinkbacitracin 40 mg/kg | ./. | | 2,59 | 97 | | |
| Kormogrisin 40 mg/kg | 1,37 | 98 | 2,49 | 93 | 2,10 | 94 |
| Nourseothricin 10 mg/kg | 1,34 | 95 | 2,58 | 96 | 2,11 | 95 |
| Nourseothricin 40 mg/kg | 1,74 | 124 | 2,65 | 99 | 2,31 | 104 |
| Kontrolle | 1,40 | 100 | 2,67 | 100 | 2,94 | 100 |

Tabelle 13: Wirkung verschiedener Ergotropika auf den
Futteraufwand bei Schweinen in Bodenhaltung
(kg/kg Zunahme)

| Lebendmasse-Abschnitt | 8 bis 45 kg | | 45 bis 120 kg | | 8 bis 120 kg | |
|---|---|---|---|---|---|---|
| (Ergotropikum) | (g) | (%) | (g) | (%) | (g) | (%) |
| Zinkbacitracin 150 mg/kg | 2,61 | 90 | ./. | | 3,58 | 91 |
| Zinkbacitracin 40 mg/kg | ./. | | 4,18 | 95 | | |
| Kormogrisin 40 mg/kg | 2,45 | 85 | 4,38 | 99 | 3,63 | 93 |
| Nourseothricin-Hydrochlorid 10 mg/kg | 2,66 | 92 | 4,28 | 97 | 3,74 | 95 |
| Nourseothricin-Hydrochlorid 40 mg/kg | 2,96 | 102 | 4,26 | 96 | 3,84 | 98 |
| Kontrolle | 2,89 | 100 | 4,42 | 100 | 3,92 | 100 |

Tabelle 14: Wirkung gestaffelter Nourseothricin-Zusätze (Myzeladsorbat) auf die durchschnittliche tägliche Lebendmasse-Zunahme (g/Tier) bei Schweinen in Bodenhaltung; Versuchsdauer 70 Tage

| Versuch | Kontrolle | | Nourseothricin-Wirkstoff (mg/kg Futter) | | | | | |
| | | | 7 | | 14 | | 21 | |
| | (g) | (%) | (g) | (%) | (g) | (%) | (g) | (%) |
| A | 479 | 100 | 496 | 104 | 548 | 114 | 580 | 123 |
| | (104) | | (140) | | (41) | | (118) | |
| B | 584 | 100 | 564 | 97 | 594 | 102 | 616 | 106 |
| | (85) | | (92) | | (118) | | (507) | |

(Zahlenwerte in Klammern ≙ Streuung der Meßwerte)

Tabelle 15: Wirkung gestaffelter Nourseothricin-Zusätze
(Myzeladsorbat) auf die durchschnittliche
tägliche Futteraufnahme (kg Futter/Tier) bei
Schweinen in Bodenhaltung; Versuchsdauer 70 Tage

| Versuch | Kontrolle | | Nourseothricin-Wirkstoff (mg/kg Futter) | | | | | |
| | | | 7 | | 14 | | 21 | |
| | (g) | (%) | (g) | (%) | (g) | (%) | (g) | (%) |
| A | 1,33 | 100 | 1,38 | 104 | 1,56 | 117 | 1,55 | 117 |
| B | 1,72 | 100 | 1,66 | 97 | 1,62 | 94 | 1,68 | 98 |

Tabelle 16: Wirkung gestaffelter Nourseothricin-Zusätze
(Myzeladsorbat) auf den durchschnittlichen
Futteraufwand (kg/kg Zunahme) bei Schweinen
in Bodenhaltung; Versuchsdauer 70 Tage

| Versuch | Kontrolle | | Nourseothricin-Wirkstoff (mg/kg Futter) | | | | | |
| | | | 7 | | 14 | | 21 | |
| | (g) | (%) | (g) | (%) | (g) | (%) | (g) | (%) |
| A | 2,78 | 100 | 2,78 | 100 | 2,85 | 102 | 2,63 | 95 |
| B | 2,94 | 100 | 2,94 | 100 | 2,75 | 93 | 2,73 | 93 |

Tabelle 17: Wirkung gestaffelter Nourseothricin-Zusätze (Myzeladsorbat) auf die Tiergesundheit und die Lebendmasse-Entwicklung bei Kälbern (9 Tiere/Gruppe)

| Nourseothricin-Wirkstoff (mg/Tier/Tag) | 0,0 | 17,5 | 35,0 | 52,5 |
|---|---|---|---|---|
| Anfangmasse (kg) | 55 | 53 | 53 | 55 |
| Durchfalltage | 58 | 17 | 11 | 9 |
| Verendungen | 1 | 0 | 0 | 0 |
| Lebendmasse-Entwicklung (g/Tier/Tage) | | | | |
| 1. bis 4. Lebendwoche | 488 (100%) | 552 (113%) | 611 (125%) | 587 (120%) |
| 1. bis 10. Lebendwoche | 663 (100%) | 708 (107%) | 742 (113%) | 729 (110%) |

Patentansprüche

1. Verfahren zur Herstellung von Nourseothricin, seiner Salze und Adsorbate durch Züchtung eines Nourseothricinbildenden Streptomycetenstammes unter submersen aeroben Kulturbedingungen in einem geeignete Kohlenstoff- und Stickstoffquellen sowie Mineralsalze enthaltenden Nährmedium und anschließende Wirkstoffisolierung,
dadurch gekennzeichnet,
daß zur Hauptkultur erforderlichenfalls Hemmstoffe der Atmungskette vom Typ der Alkaliazide, des Brenzkatechins und/oder der Ethylisoamylbarbitursäure und/oder Hemmstoffe des Aminosäuretransports vom Typ wasserlöslicher Zinksalze und/oder ß-Alanin zugesetzt werden; daß eine geregelte Hitzesterilisation des Nährmediums für die Hauptkultur in Anwesenheit Phosphat-fällender Substanzen, insbesondere von Salzen des Zink(II), Eisen(III), Aluminium(III), Calcium (II), Mangan(II) oder Magnesium(II), neben Sulfationen und Calciumcarbonat und erforderlichenfalls unter vorherigem Zusatz von Lauge durchgeführt wird, wobei nach Beendigung der Sterilisation ein pH-Wert im Bereich von 7,2 bis 8,2 erreicht wird; daß während der Fermentation eine ratenlimitierte Phosphatversorgung im Bereich von 0,1 bis 10 mg Phosphat-Phosphor/l · h gewährleistet, ein pH-Wert im Bereich von pH 5 bis 6,5 eingehalten und ein $O_2$-Partialdruck vom 30 % bis 80 %, vorzugsweise von 40 % bis 60 % eingestellt wird sowie weitere Substratlimitationen, insbesondere von Ammonium-Stickstoff und Glucose bzw. Oligo- oder Polysacchariden bei Aufrechterhaltung eines Kohlenstoff-Stickstoff-Konzentrationsverhältnisses von 5 bis 15 g Glucose-Einheit/l zu 0,015 bis 0,2 Ammonium-Stickstoff/l gewährleistet werden und daß das Antibiotikum durch Zugabe eines Adsorbens, beispielsweise Bentonit, zur Kulturlösung als Nourseothricin-Adsorbat oder daß es durch Adsorption aus dem Kulturfiltrat an schwach saure Kationenaustauscher, Elution mit verdünnten mehrbasischen Säuren, beispielsweise mit Schwefelsäure, Oxalsäure oder Phosphorsäure, durch anschließende Konzentrierung, Neutralisation und Reinigung sowie durch Ausfällung in Methanol bzw. Trocknung der Salze der entsprechenden Säure isoliert wird.

2. Verfahren nach Anspruch 1, dadurch gekennzeichnet, daß die Laugedosierrate zur pH-Sollwert-Regelung, zur Steuerung der Zugabe von Kohlenstoff- oder Stickstoffquellen und/oder von Substanzen mit Effektorwirkung sowie zur Steuerung der Phosphatdosierung verwendet wird.

3. Verfahren nach Anspruch 1 oder 2, dadurch gekennzeichnet, daß die Reaktionswärme als Steuergröße herangezogen wird.

4. Verfahren nach einem der Ansprüche 1 bis 3, dadurch gekennzeichnet, dass die geregelte Hitzesterilisation durch Steuerung des Wärmeeintrages auf der Basis einer Sterilisationsfunktionale geführt wird.

E

FIG. 1

FIG. 2

FIG. 3

Diagramm 1

Diagramm 2

FIG.4

3/6

0136256

GLC NH₄-N [g/l] [g/l] ph

pH-Wert

Stickstoffkonzentration

Glukosekonzentration(GLC)

t[h]

Diagramm 3

FIG.5

NH$_4$OH-Rate  PO$_4$-P  pO$_2$
[ml/m$^3$·h] [mg/l] [‰]

pO$_2$-Wert

spezifische NH$_4$OH-Rate

PO$_4$-P - Konzentration

t[h]

0136256

**Abluft**

$CO_2$ $O_2$ $Q_R$
[%] [%] [KW/m$^3$]

Diagramm 4

FIG.6

spezifische Reaktionswärme ($Q_R$)

$O_2$

$CO_2$

5/6

0136256

t[h]

kp · μ
[1/h] [1/h]

Diagramm 5

FIG. 7

spezifische
Produktbildungsrate kp

spezifische
Wachstumsrate μ

0        50       100      150      t[h]

6/6

0136256